(19)

Europäisches Patentamt
European Patent Office
Office européen des brevets

(11)    **EP 3 052 943 B1**

(12)    **EUROPEAN PATENT SPECIFICATION**

(45) Date of publication and mention
of the grant of the patent:
**20.11.2019  Bulletin 2019/47**

(21) Application number: **14851360.9**

(22) Date of filing: **03.10.2014**

(51) Int Cl.:
*G01N 33/68* (2006.01)        *A61K 35/12* (2015.01)
*A61P 1/00* (2006.01)        *A61P 19/02* (2006.01)
*A61P 37/00* (2006.01)

(86) International application number:
**PCT/AU2014/000951**

(87) International publication number:
**WO 2015/048842 (09.04.2015 Gazette 2015/14)**

(54) **BIOMARKERS FOR CELL THERAPY**

BIOMARKER FÜR EINE ZELLTHERAPIE

BIOMARQUEURS POUR THÉRAPIE CELLULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **04.10.2013  AU 2013903840**

(43) Date of publication of application:
**10.08.2016  Bulletin 2016/32**

(73) Proprietor: **Cell Ideas Pty Ltd.
Pymble, NSW 2073 (AU)**

(72) Inventor: **HERBERT, Ben
North Epping, NSW 2121 (AU)**

(74) Representative: **Huenges, Martin
Maiwald Patentanwalts- und
Rechtsanwaltsgesellschaft mbH
Elisenhof
Elisenstraße 3
80335 München (DE)**

(56) References cited:
**EP-A1- 2 639 296        WO-A1-98/51317
WO-A1-03/024215        WO-A1-2010/062663
WO-A1-2013/023233        WO-A1-2013/050453
US-A1- 2011 212 104        US-A1- 2011 293 577
US-A1- 2014 248 638**

- **YPE P. DE JONG ET AL: "Development of chronic colitis is dependent on the cytokine MIF", NATURE IMMUNOLOGY, vol. 2, no. 11, 1 November 2001 (2001-11-01), pages 1061-1066, XP055332397, ISSN: 1529-2908, DOI: 10.1038/ni720**
- **JONES E1 ET AL: "Mesenchymal stem cells in rheumatoid synovium: enumeration and functional assessment in relation to synovial inflammation level", ANNALS OF THE RHEUMATIC DISEASES, BRITISH MEDICAL ASSOCIATION, GB, vol. 69, no. 2, 1 February 2010 (2010-02-01), pages 450-457, XP009193981, ISSN: 0003-4967, DOI: 10.1136/ARD.2008.106435**
- **MOBASHERI, A. ET AL.: 'Biomarkers of Osteoarthritis: A Review of Recent Research Progress on Soluble Biochemical Markers, Published Patents and Areas for Future Development' RECENT PATENTS OF BIOMARKERS vol. 1, 2011, pages 25 - 43, XP055332388**
- **HOES, J.N. ET AL.: 'Changes in macrophage inhibitory factor correlate with changes in bone mineral density in glucocorticoid-treated patients with rheumatoid arthritis' RHEUMATOLOGY vol. 50, 2011, pages 1921 - 1924, XP055332391**
- **KASAMA, T. ET AL.: 'Serum macrophage migration inhibitory factor levels are correlated with response to tocilizumab therapy in patients with rheumatoid arthritis' RHEUMATOLOGY INTERNATIONAL vol. 34, no. 3, 14 May 2013, pages 429 - 433, XP055332394**

**(Cont. next page)**

- DE JONG, Y.P. ET AL.: 'Development of chronic colitis is dependent on the cytokine MIF' NATURE IMMUNOLOGY vol. 2, no. 11, 2001, pages 1061 - 1066, XP055332397
- REN, Y. ET AL.: 'Up-regulation of macrophage migration inhibitory factor in infants with acute neonatal necrotizing enterocolitis' HISTOPATHOLOGY vol. 46, no. 6, 2005, pages 659 - 667, XP055332400
- PAK, J.: 'Regeneration of human bones in hip osteonecrosis and human cartilage in knee osteoarthritis with autologous adipose-tissue-derived stem cells: a case series' JOURNAL OF MEDICAL CASE REPORTS vol. 5, no. 296, 2011, pages 1 - 8, XP021106048
- LANZONI, G. ET AL.: 'Inflammatory bowel disease: Moving toward a stem cell -based therapy' WORLD JOURNAL OF GASTROENTEROLOGY vol. 14, no. 29, 2008, pages 4616 - 4626, XP055127973

## Description

## Field

The invention relates to methods for monitoring disease progression in a patient having mesenchymal stem cell therapy for an inflammatory condition, the method comprising determining the level of a biomarker MIF. In particular embodiments the patient is undergoing treatment of the inflammatory condition and the method comprises monitoring the level of MIF an optionally further biomarkers to monitor disease progression, for example to assist the clinician in optimising the treatment regimen. In particular embodiments the invention relates to methods of monitoring the effectiveness of autologous or allogeneic cell therapy of a patient having a condition characterised by cartilage damage or degeneration, such as OA, for example in order to assist a practitioner in determining an appropriate time to administer a further dose of cells. The invention also provides kits and components for use in the methods.

## Background

Inflammation and inflammatory conditions present a serious health problem in the general population, and even more specifically in an aging population where chronic inflammatory conditions can present ongoing debilitating and degenerating mobility and pain. Inflammation may arise as a response to an injury or abnormal stimulation caused by a physical, chemical, or biologic agent. The term "inflammatory" when used in reference to a disorder refers to a pathological process that is caused by, resulting from, or resulting in inflammation that is inappropriate or which does not resolve in the normal manner. Inflammatory disorders may be systemic or localized to particular tissues or organs.

Osteoarthritis (OA) is an idiopathic, incurable chronic and debilitating musculoskeletal disease and is reported by more than 1.4 million people in Australia. OA onset is most closely associated with ageing and the key observations are cartilage changes and pain. It is classically referred to as a non-inflammatory disease but it is increasingly evident that inflammation plays a major role in OA disease progression. Patients with OA are typically managed with non-steroidal anti-inflammatory drugs (NSAIDs) and analgesics to alleviate OA symptoms and to control the pain in affected joints. Currently, when NSAIDs and also corticosteroid therapy are no longer beneficial, the usual treatment is total joint arthroplasty. This poses a significant problem for patients who are 30-60 years old. Many orthopaedic surgeons are hesitant to perform a joint replacement on people under 50 because the implant is unlikely to last their lifetime.

In recent years there has been a shift in medical research towards innovative regenerative treatments for a variety of diseases. In joint diseases such as arthritis, a number of research groups have used animal models of OA to explore the use of adult mesenchymal stem cells (MSCs) as a potential regenerative therapy. In animal models of acute and chronic cartilage damage, treatment with MSCs produces meniscal and hyaline cartilage regeneration and reductions in OA-like disease progression, cartilage loss, osteophyte formation and subchondral thickening. These cells have also been demonstrated to have significant anti-inflammatory and immunomodulatory effects through the secretion of bioactive factors. Adipose tissue is a particularly attractive source of cells for therapeutic purposes as it contains 500-1000 times more MSCs per gram than bone marrow. Along with an abundance of MSCs, adipose tissue also comprises immune cells, vascular smooth muscle cells, endothelial cells, and pericytes, which collectively are termed the stromal vascular fraction (SVF). The ability to obtain large quantities of adipose tissue through standard liposuction techniques and the ability to rapidly isolate the SVF, allows for in-clinic same-day cell therapy procedures.

For example, International patent application PCT/AU2009/001070 (WO2010/020005) entitled "Therapeutic methods using adipose tissue-derived cell suspensions comprising adipocytes" and Australian Patent Application No. 2009201915, the contents of both of which are incorporated herein by cross-reference, describe autologous adipose-derived cell therapy for the treatment of musculoskeletal conditions, including OA.

Australian Patent Application No. 2013204930 entitled "Therapeutics using multiple injections of cells" described that it had surprisingly been identified that an autologous adipose tissue-derived cell suspension may be frozen and retrieved for subsequent administration as a course of planned injections to provide an improved therapeutic outcome compared to a single dose. Therein it was described that surprisingly such frozen cells may be used without the need for culturing the cells after retrieval from frozen storage. As described in AU2013204930 this allowed the inventors to develop improved methods of treating osteoarthritis, and various other conditions, including pain, by administering to the subject a course of treatment comprising multiple doses over time of an autologous adipose tissue-derived cell suspension comprising adipose tissue-derived non-adipocyte cells, wherein a first dose comprises a portion of a freshly prepared cell suspension and a subsequent dose or doses comprise a portion of the cell suspension that has been stored frozen. US 2011/0212104 relates to biomarkers associated with inflammatory bowel disease and methods for using such biomarkers for diagnosing, assessing and monitoring disease progression. US 2014/0248638 relates to the recognition that the specific oxMIF form of MIF is useful as diagnostic marker in MIF-related diseases. Jong Y. P. et al. (Nature Immunology 2(11); 2001) reports that the development of chronic colitis is dependent on the cytokine MIF. EP 2 639 296 A1 relates to a mammalian stem cell suspension containing mammalian stem cells and at least one polysaccharide. WO 98/51317 concerns osteoarthritis cartilage regeneration using human mesenchymal stem cells. US 2011/029357 discloses therapeutic methods using adipose tissue-derived cell suspensions comprising adipocytes. WO2013/023233 relates to a method for diagnosing acute coronary syndrome (ACS) in a subject comprising measuring MIF concentration and diagnosing ACS when the subject plasma MIF concentration is greater than a reference plasma MIF concentration. Jones *et al.* reports on the enumeration and functional assessment of mesenchymal stem cells in rheumatoid synovium.

There remains a need for additional methods to assist in the treatment of patients having inflammatory conditions, OA and related disorders, as well as other conditions in which cartilage damage or degeneration is involved.

## Summary of Invention

The inventor recognised that it would be advantageous for the treating physician to have a method to assist them in determining the progression of an inflammatory condition in a patient, for example to assist in guiding decisions concerning an appropriate time at which to administer a therapeutic dose to the patient, for example a dose of MSCs, or of an adipose tissue-derived cell suspension, which may be an autologous or an allogeneic cell suspension, to the patient, particularly a method that is independent of the patient's subjective assessment of their own condition such as self-reporting of pain scores or discomfort levels.

As described herein methods are provided for the use of biomarkers to assess the progression of an inflammatory condition and to assist in identifying appropriate treatment times for mesenchymal cell-based therapy of conditions characterised by inflammation. The inventor has identified that macrophage migration inhibitory factor (MIF) is detectable in the serum of patients undergoing mesenchymal stem cell treatment for inflammatory conditions, such as OA, neurodegenerative disease, and ulcerative colitis, and that levels of detectable MIF correlate with treatment outcome, such as stabilisation or improvement of the inflammatory condition. In a particular example, exemplified herein in the treatment of OA, levels of detectable MIF correlate with treatment outcome, such as reduced cartilage degradation. MIF is an inflammatory cytokine that stimulates the degradation of damaged tissue.

The inventor has also identified that CTX-II, a C-terminal telopeptide of type II collagen, is detectable in the serum and in the urine of patients undergoing treatment for OA and that levels of detectable CTX-II correlate with cartilage degradation. The serum levels of MIF correlate with reduced tissue degradation observed after MSC treatment, for example in OA, reduced serum MIF correlates with reduced urinary CTX II, which is a marker of cartilage degradation.

The inventor has also identified that COMP is an additional cartilage specific breakdown product that is well correlated with OA. It increases (in serum) during the progression of disease. As with CTX, the examples herein demonstrate a post-treatment stabilisation or slight decrease of this marker.

Accordingly, in an aspect of the invention there is provided a method for monitoring disease progression in a patient having mesenchymal stem cell therapy for an inflammatory condition, the method comprising determining the level of a biomarker MIF in at least a first and a second biological sample from said patient, wherein a change in the level of said biomarker in said second compared to said first biological sample is indicative of disease progression wherein (i) an increase in the detectable level of said biomarker in a second compared to a first biological sample is indicative of pathological progression, or deterioration, of the inflammatory condition; or (ii) a decrease in the detectable level of said biomarker in a second compared to a first biological sample is indicative of stabilisation of or improvement of the inflammatory condition, and wherein said therapy comprises

(i) a course of treatment comprising multiple doses over time of a cell suspension comprising mesenchymal stem cells (MSCs); or (ii) a course of treatment comprising multiple doses over time of an autologous adipose tissue-derived cell suspension comprising adipose tissue-derived non-adipocyte cells, wherein a first dose comprises a portion of a freshly prepared cell suspension and a subsequent dose or doses comprise a portion of said cell suspension that has been stored frozen; or (iii) a course of treatment comprising multiple doses over time of an allogeneic adipose tissue-derived cell suspension comprising adipose tissue-derived non-adipocyte cells, wherein all doses comprise a cell suspension that has been stored frozen. In this terminology disease progression may be that the condition has worsened, stabilised or improved.

Herein described is a method of treating an inflammatory condition in a subject requiring said treatment, the method comprising administering to said subject a cell suspension comprising mesenchymal stem cells (MSCs), wherein the method further comprises determining the level of a biomarker MIF in at least a first and a second biological sample from said patient.

Also described herein is method of treating an inflammatory condition in a subject requiring said treatment, the method comprising administering to said subject a course of treatment comprising multiple doses over time of a cell suspension comprising mesenchymal stem cells (MSCs), wherein the method further comprises determining the level of a biomarker MIF in at least a first and a second biological sample from said patient.

The disclosure also refers to a method of treating an inflammatory condition in a subject requiring said treatment, the method comprising administering to said subject a course of treatment comprising multiple doses over time of an autologous adipose tissue-derived cell suspension comprising adipose tissue-derived non-adipocyte cells, wherein a first dose comprises a portion of a freshly prepared cell suspension and a subsequent dose or doses comprise a portion of said cell suspension that has been stored frozen, the method further comprising determining the level of a biomarker MIF in at least a first and a second biological sample from said patient.

In a further aspect of the disclosure there is provided a method of treating an inflammatory condition in a subject requiring said treatment, the method comprising administering to said subject a course of treatment comprising multiple doses over time of an allogeneic adipose tissue-derived cell suspension comprising adipose tissue-derived non-adipocyte cells, wherein all doses comprise a cell suspension that has been stored frozen, the method further comprising determining the level of a biomarker MIF in at least a first and a second biological sample from said patient.

In a further aspect the disclosure provides a method of treating an inflammatory condition in a subject requiring said treatment, the method comprising the steps of administering to said patient a first dose of an autologous adipose tissue-derived cell suspension comprising adipose tissue-derived non-adipocyte cells, wherein the first dose comprises a portion of a freshly prepared cell suspension, determining the level of the biomarker MIF in at least a first and a second biological sample from said patient, wherein the first biological sample is a baseline sample from said patient prior to commencement of said treatment and the second biological sample is a sample from said patient after said first dose, wherein if the level of the biomarker MIF is approximately the same in the second sample compared to the first sample, administering a further dose of the autologous adipose tissue-derived cell suspension, wherein the further dose comprises a portion of the cell suspension that had been administered to the patient at the commencement of the treatment, the portion having been stored frozen prior to use.

In a further aspect the disclosure provides a method of treating an inflammatory condition in a subject requiring said treatment, the method comprising the steps of administering to said patient a first dose of an allogeneic adipose tissue-derived cell suspension comprising adipose tissue-derived non-adipocyte cells, determining the level of the biomarker MIF in at least a first and a second biological sample from said patient, wherein the first biological sample is a baseline sample from said patient prior to commencement of said treatment and the second biological sample is a sample from said patient after said first dose, wherein if the level of the biomarker MIF is approximately the same in the second sample compared to the first sample, administering a further dose of the allogeneic adipose tissue-derived cell suspension, wherein all doses of cell suspension have been stored frozen prior to use.

In a further aspect the disclosure provides a method of treating an inflammatory condition in a subject requiring said treatment, the method comprising the steps of administering to said patient a first dose of an autologous adipose tissue-derived cell suspension comprising adipose tissue-derived non-adipocyte cells, wherein the first dose comprises a portion of a freshly prepared cell suspension, determining the level of the biomarker MIF in at least a first and a second biological sample from said patient, wherein both the first and the second biological samples are obtained from said patient after said first dose, wherein if an increase in the level of the biomarker MIF is determined in the second sample compared to the first sample, administering a further dose of the autologous adipose tissue-derived cell suspension, wherein the further dose comprises a portion of the cell suspension that had been administered to the patient at the commencement of the treatment, the portion having been stored frozen prior to use.

In a further aspect the disclosure provides a method of treating an inflammatory condition in a subject requiring said treatment, the method comprising the steps of administering to said patient a first dose of an allogeneic adipose tissue-derived cell suspension comprising adipose tissue-derived non-adipocyte cells, determining the level of the biomarker MIF in at least a first and a second biological sample from said patient, wherein both the first and the second biological samples are from said patient after said first dose, wherein if an increase in the level of the biomarker MIF is determined in the second sample compared to the first sample, administering a further dose of the allogeneic adipose tissue-derived cell suspension, wherein all doses of cell suspension have been stored frozen prior to use.

In an embodiment the MSCs are selected from autologous cells, allogeneic cells, cord blood cells, and expanded cord blood cells, or a mixture thereof.

In an embodiment the inflammatory condition is a condition characterised by or associated with cartilage damage or degeneration. In an embodiment the inflammatory condition is selected from the group consisting of osteoarthritis, rheumatoid arthritis and inflammatory bowel disease. In an embodiment the inflammatory bowel disease is ulcerative colitis.

In an embodiment the method of monitoring or treating an inflammatory condition, wherein the inflammatory condition is selected from OA or a condition characterised by or associated with cartilage damage or degeneration, further comprises determining the level of at least a second biomarker selected from CTX-II and COMP in said biological sample or samples.

In a further aspect the invention provides a method for monitoring disease progression in a patient having a condition characterised by inflammation and tissue degradation, such as cartilage damage or degeneration, the method comprising determining the level of the biomarker MIF and optionally of at least one biomarker selected from COMP and CTX-II in at least a first and a second biological sample from said patient, wherein a change in the level of said biomarker in said second compared to said first biological sample is indicative of disease progression. In this terminology disease progression may be that the condition has worsened, stabilised or improved.

In a further aspect of the disclosure there is provided a method of treating a condition characterized by cartilage damage or degeneration in a subject requiring said treatment, the method comprising administering to said subject a cell suspension comprising mesenchymal stem cells (MSCs), the method further comprising determining the level of at least one biomarker selected from COMP, CTX-II and MIF in at least a first and a second biological sample from said patient.

In a further aspect of the disclosure there is provided a method of treating a condition characterized by cartilage damage or degeneration in a subject requiring said treatment, the method comprising administering to said subject a course of treatment comprising multiple doses over time of a cell suspension comprising mesenchymal stem cells (MSCs), the method further comprising determining the level of at least one biomarker selected from COMP, CTX-II and MIF in at least a first and a second biological sample from said patient.

In a further aspect of the disclosure there is provided a method of treating a condition characterized by cartilage damage or degeneration in a subject requiring said treatment, the method comprising administering to said subject a course of treatment comprising multiple doses over time of an autologous adipose tissue-derived cell suspension comprising adipose tissue-derived non-adipocyte cells, wherein a first dose comprises a portion of a freshly prepared cell suspension and a subsequent dose or doses comprise a portion of said cell suspension that has been stored frozen, the method further comprising determining the level of at least one biomarker selected from COMP, CTX-II and MIF in at least a first and a second biological sample from said patient.

In a further aspect of the disclosure there is provided a method of treating a condition characterized by cartilage damage or degeneration in a subject requiring said treatment, the method comprising administering to said subject a course of treatment comprising multiple doses over time of an allogeneic adipose tissue-derived cell suspension comprising adipose tissue-derived non-adipocyte cells, wherein all doses comprise a cell suspension that has been stored frozen, the method further comprising determining the level of at least one biomarker selected from COMP, CTX-II and MIF in at least a first and a second biological sample from said patient.

In an embodiment, where the cell suspension is an allogeneic cell suspension, the patient is a non-human animal, such as a cat, dog or horse.

In an embodiment the condition characterised by cartilage damage or degeneration is a chronic condition. In an embodiment the condition characterised by cartilage damage or degeneration is arthritis. In an embodiment the condition characterised by cartilage damage or degeneration is osteoarthritis (OA) or rheumatoid arthritis (RA).

In an embodiment the condition characterised by cartilage damage or degeneration is an acute condition, such as injury or trauma to a cartilage.

In an embodiment the method comprises determining the level of MIF and at least one of COMP and CTX-II in at least a first and a second biological sample from said patient.

In an embodiment the method comprises determining the level of COMP, CTX-II and MIF in at least a first and a second biological sample from said patient.

The first and second biological samples may be any two samples from the patient separated in time of collection. In an embodiment the first biological sample is a baseline sample from said patient prior to commencement of a treatment for the condition. In an embodiment the second biological sample is a sample from said patient after commencement of a treatment for the condition. In an embodiment the second biological sample is a sample from said patient one week to twelve months after commencement of a treatment for the condition. In an embodiment both the first and the second biological samples are samples from the patient after commencement of the treatment.

In an embodiment the first biological sample is a sample from said patient prior to commencement of a treatment for said condition and the second biological sample is a sample from said patient after commencement of the treatment, wherein if the determined level of said at least one biomarker in said first and said second samples is approximately the same, administering a further dose of the cell suspension.

In an embodiment the method further comprises determining the level of said biomarker in additional biological samples from said patient, such as a third, fourth, fifth, etc biological sample, wherein each of said biological samples is obtained from said patient at different times before and or after commencement of a treatment for the condition. For example, a first biological sample may be a sample from said patient prior to commencement of a treatment for the condition, and a second and subsequent biological samples may be a sample or samples from said patient at approximately monthly intervals after commencement of the treatment for the condition.

In one embodiment the biological sample is serum. In one embodiment the biological sample is urine. In one embodiment the biomarker is CTX-II and the biological sample is urine or serum. In one embodiment the biomarker is MIF and the biological sample is serum. In one embodiment the biomarker is COMP and the biological sample is serum.

In an embodiment the method comprises the steps of administering to said patient a first dose of an autologous adipose tissue-derived cell suspension comprising adipose tissue-derived non-adipocyte cells, wherein the first dose comprises a portion of a freshly prepared cell suspension, determining the levels of MIF and optionally further of one or more of the biomarkers COMP and CTX-II in at least a first and a second biological sample from said patient, wherein the first biological sample is a baseline sample from said patient prior to commencement of said treatment and the second biological sample is a sample from said patient after said first dose, wherein if the level of MIF and optionally at least one of the biomarkers COMP and CTX-II is approximately the same in the second sample compared to the first sample, administering a further dose of the autologous adipose tissue-derived cell suspension, wherein the further dose comprises a portion of the cell suspension that had been administered to the patient at the commencement of the treatment, the portion having been stored frozen prior to use.

In an embodiment the course of treatment comprises the steps of administering to said patient a first dose of an allogeneic adipose tissue-derived cell suspension comprising adipose tissue-derived non-adipocyte cells, determining the levels of MIF and optionally of one or more of the biomarkers COMP and CTX-II in at least a first and a second biological sample from said patient, wherein the first biological sample is a baseline sample from said patient prior to commencement of said treatment and the second biological sample is a sample from said patient after said first dose, wherein if the level of MIF and optionally of at least one of the biomarkers COMPand CTX-II is approximately the same in the second sample compared to the first sample, administering a further dose of the allogeneic adipose tissue-derived cell suspension, wherein all doses of cell suspension have been stored frozen prior to use.

In an embodiment the course of treatment comprises the steps of administering to said patient a first dose of an autologous adipose tissue-derived cell suspension comprising adipose tissue-derived non-adipocyte cells, wherein the first dose comprises a portion of a freshly prepared cell suspension, determining the levels of MIF and optionally one or more of the biomarkers COMP and CTX-II in at least a first and a second biological sample from said patient, wherein both the first and the second biological samples are obtained from said patient after said first dose, wherein if an increase in the level of MIF and optionally at least one of the biomarkers COMP and CTX-II is determined in the second sample compared to the first sample, administering a further dose of the autologous adipose tissue-derived cell suspension, wherein the further dose comprises a portion of the cell suspension that had been administered to the patient at the commencement of the treatment, the portion having been stored frozen prior to use.

In an embodiment the course of treatment comprises the steps of administering to said patient a first dose of an allogeneic adipose tissue-derived cell suspension comprising adipose tissue-derived non-adipocyte cells, determining the levels of MIF and optionally one or more of the biomarkers COMP and CTX-II in at least a first and a second biological sample from said patient, wherein both the first and the second biological samples are from said patient after said first dose, wherein if an increase in the level of MIF and optionally at least one of the biomarkers COMP and CTX-II is determined in the second sample compared to the first sample, administering a further dose of the allogeneic adipose tissue-derived cell suspension, wherein all doses of cell suspension have been stored frozen prior to use.

In an embodiment, where the patient is a human, the method of the present invention is performed by a medical practitioner or by a person or persons under the supervision of a medical practitioner, or by a combination thereof.

In an embodiment, where the patient is a human, all steps of the method are performed by or under the supervision of a registered medical practitioner having prime responsibility for the clinical care of said subject throughout said method.

In an embodiment, one or more step(s) of the method is conducted by a person or persons under the supervision of said medical practitioner. In one embodiment, the collective steps of the method are performed by a plurality of individuals.

In an embodiment, the collective steps of the method are performed at multiple locations. In one embodiment, the step of obtaining a biological sample from said subject is conducted at a different location to one or more of the steps of administering a dose, preparing the biological sample for assay, or determining the level of at least one biomarker.

The summary of the invention described above is not limiting and other features and advantages of the invention will be apparent from the following detailed description of the preferred embodiments, as well as from the claims.

**Brief Description of Drawings**

Figure 1: ICOAP total pain scores for placebo and treatment groups throughout the trial. Data is presented as the average $\pm$ standard error of the mean (SEM).

Figure 2: Baseline QMetrics MRI assessment of cartilage damage in the test knee of trial participants.

Figure 3: Creatinine corrected CTX-II levels measured in the urine of trial participants. Data is presented as the average $\pm$ standard error of the mean (SEM).

Figure 4: Circulating serum MIF levels in trial participants at baseline and 6 months. Data is presented as the average $\pm$ SEM of the $\log_2$ fluorescence val-

ues.

Figure 5: Stratification of CTX-II data by baseline MRI derived OA grades in the treatment (A) and placebo (B) groups. Data is presented as the average ± standard error of the mean (SEM).

Figure 6: Baseline MIF levels before the second MSC treatment in 2011 and MIF at 2 months and 5 months post-treatment in two boys with a neurodegenerative disease.

Figure 7: Results of assessment by therapists of two boys with a neurodegenerative disease after treatment with MSCs, showing their scores recorded for various neurological and physical tasks.

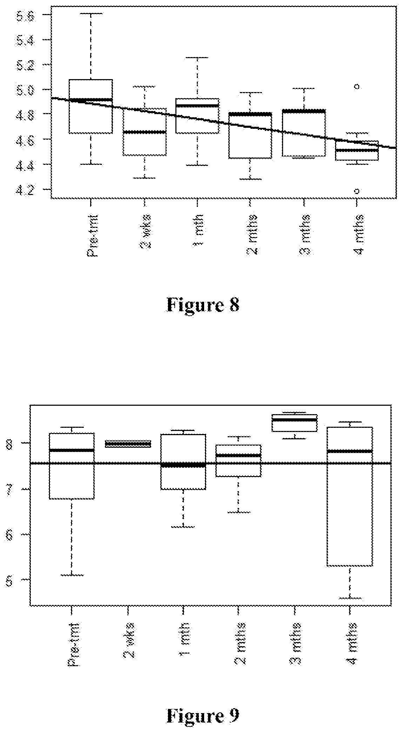

Figure 8: Circulating serum MIF levels in HiQCell patients at baseline and out to 4 months post-treatment. Normalised Macrophage Migration Inhibitory Factor (MIF).The y-value on the plot is the predicted log2(Val) from a mixed effect model. The line drawn over the box plots was determined from the mixed model's offset -1.388 and slope -0.06848. Statistically, the slope is significantly different from 0 with a p-value of 0.001. A mixed linear model was used where patient IDs formed a random effect, with 11 levels, and Time a fixed factor with 8 levels. Val represents the MIF assay reading: log2(Val)~Time + (1|ID).

Figure 9: Boxplot of creatinine corrected urinary CTX levels HiQCell joint registry participants at baseline and out to 4 months post-treatment. Normalised CTX from time trend mixed model. The y-value on the plot is the predicted log2(Val) from a mixed effect model. The line drawn over the box plots was determined from the mixed model's offset -1.173 and slope 0.01265. Statistically, the slope is NOT significantly different from 0 with a p-value of 0.983. A mixed linear model was used where patient IDs formed a random effect, with 12 levels, and Time a fixed factor with 8 levels. Val represents the ELISA absorbance reading. log2(Val)~Time + (1|ID).

Figure 10: Boxplot of serum COMP levels HiQCell joint registry participants at baseline and out to 4 months post-treatment. Normalised COMP from time trend mixed model. The y-value on the plot is the predicted log2(Val) from a mixed effect model. Statistically, the slope is NOT significantly different from 0 with a p-value of 0.285. A mixed linear model was used where patient IDs formed a random effect, with 12 levels, and Time a fixed factor with 8 levels. Val represents the ELISA absorbance reading.

$$log2(Val) \sim Time + (1|ID).$$

Figure 11: Baseline serum MIF levels before MSC treatment in 2014 and MIF at 6 weeks and 12 weeks post-treatment in two adults with ulcerative colitis. Two adults with ulcerative colitis were treated once in 2013 with culture expanded allogeneic umbilical cord blood derived MSCs. Cells were administered intravenously.

## Abbreviations

SVF as used herein is an abbreviation for stromal vascular fraction.

CTX-II as used herein is an abbreviation for C-telopeptide of type II collagen.

MIF as used herein is an abbreviation for macrophage migration inhibitory factor.

OA as used herein is an abbreviation for osteoarthritis.

RA as used herein is an abbreviation for rheumatoid arthritis.

NSAIDs as used herein is an abbreviation for non-steroidal anti- inflammatory drugs.

ICOAP as used herein is an abbreviation for Intermittent and Continuous Osteoarthritis Pain.

OSCARS as used herein is an abbreviation for Osteoarthritis Stem Cell Advanced Research Study.

OARSI as used as used herein is an abbreviation for Osteoarthritis Research Society International.

MSCs as used as used herein is an abbreviation for mesenchymal stem cells.

COMP as used herein is an abbreviation for cartilage oligomeric matrix protein.

HSCs as used herein is an abbreviation for Hematopoeitic Stem Cells.

## Definitions

The term "pharmaceutically acceptable" as used herein in the context of various components relevant to the invention, such as carriers, diluents, cryopreservatives, is intended to encompass not only such components which are suitable for administration to a human subject, but also those suitable for administration to a non-human mammalian subject. In particular embodiments, the pharmaceutically acceptable component is suitable for administration to a non-human mammalian subject. In particular embodiments the pharmaceutically acceptable component is suitable for administration to a human subject. In particular embodiments, the pharmaceutically acceptable component is suitable for administration to a non-human mammalian subject and to a human subject.

Unless the context indicates otherwise, the terms disorder, condition and disease are generally used interchangeably herein.

The terms "treating", "treatment", "therapy" and the like in the context of the present specification refer to the alleviation of the symptoms and/or the underlying cause of the condition or disease, such as the inflammation or inflammatory condition, or the osteoarthritis, or the condition associated with or characterised by cartilage damage or degeneration, or tendon injury or pain. In certain embodiments a treatment will slow, delay or halt the progression of a disorder or the symptoms of the disorder or injury, or reverse the progression of the disorder or injury, at least temporarily. Hence, in the context of this invention the word "treatment" or derivations thereof such as "treating" when used in relation to a therapeutic application includes all aspects of a therapy, such as the alleviation of pain associated with the condition being treated, alleviation of the severity of the condition being treated, improvement in one or more symptoms of the condition being treated, etc. Use of the word "treatment" or derivatives thereof will be understood to mean that the subject being "treated" may experience any one or more of the aforementioned benefits.

Throughout this specification, reference to "a" or "one" element does not exclude the plural, unless context determines otherwise. Similarly, reference to "an embodiment" does not exclude the characteristic of that described embodiment applying in combination with one or more other embodiments described, unless the context determines otherwise.

The term "therapeutically effective amount" as used herein includes within its meaning a non-toxic but sufficient amount of a compound or composition for use in the invention to provide the desired therapeutic effect. The exact amount required will vary from subject to subject depending on factors such as the species being treated, the age and general condition of the subject, co-morbidities, the severity of the condition being treated, the particular agent being administered and the mode of administration and so forth. Thus, for any given case, an appropriate "effective amount" may be determined by one of ordinary skill in the art using only routine methods.

In the context of this specification, the term "comprising" means including, but not necessarily solely including. Furthermore, variations of the word "comprising", such as "comprise" and "comprises", have correspondingly varied meanings. Hence, the term "comprising" and variations thereof is used in an inclusive rather than exclusive meaning such that additional integers or features may optionally be present in a composition, method, etc. that is described as comprising integer A, or comprising integer A and B, etc.

In the context of this specification the terms "about" and "approximately" will be understood as indicating the usual tolerances that a skilled addressee would associate with the given value.

In the context of this specification, where a range is stated for a parameter it will be understood that the parameter includes all values within the stated range, inclusive of the stated endpoints of the range. For example, a range of "5 to 10" will be understood to include the values 5, 6, 7, 8, 9, and 10 as well as any sub-range within the stated range, such as to include the sub-range of 6 to 10, 7 to 10, 6 to 9, 7 to 9, etc, and inclusive of any value and range between the integers which is reasonable in the context of the range stated, such as 5.5, 6.5, 7.5, 5.5 to 8.5 and 6.5 to 9, etc. For example, a range of "10% to 30%" will be understood to include the values 10%, 11%, 12%, 13%, and all integers up to and including 30%, as well as any sub-range within the stated range, such as to include the sub-range of 10% to 15%, 12% to 18%, 20% to 30%, etc, and inclusive of any value and range between the integers which is reasonable in the context of the range stated, such as 10.5%, 15.5%, 25.5%, etc.

In the context of this specification, the terms "plurality" and "multiple" mean any number greater than one.

It is to be noted that reference herein to use of the inventive methods and compositions in treatment or therapy will be understood to be applicable to human and non-human, such as veterinary, applications. Hence it will be understood that, except where otherwise indicated, reference to a patient, subject or individual means a human or a non-human, such as an individual of any species of social, economic, agricultural or research importance including but not limited to members of the classifications of ovine, bovine, equine, porcine, feline, canine, primates, rodents, especially domesticated or farmed members of those classifications, such as sheep, cattle, horses, pigs and dogs.

Where examples of various embodiments or aspects of the invention are described herein they will generally be prefaced by appropriate terms including "such as" or "for example", or "including". It will be understood that the examples are being described as inclusive possibilities, such as for the purpose of illustration or understanding and are not, unless the context indicates otherwise, being provided as limiting.

The pharmaceutical composition referred to herein may also be referred to as a medicament, such as when intended for therapeutic use. Hence, it will be understood that where the invention is described as including the use of a composition of described components for the preparation of a pharmaceutical composition for an intended therapeutic purpose, that description equally means use for the preparation of a medicament for that intended therapeutic purpose, unless the context indicates otherwise.

To the extent that it is permitted, all references cited herein are incorporated by reference in their entirety.

Any description of documents herein, or statements herein derived from or based on those documents, is not an admission that the documents or derived statements are part of the common general knowledge of the relevant art in Australia or elsewhere.

## Description of Embodiments

In known methods for the treatment of inflammatory conditions, such as neurodegenerative disease, inflammatory bowel disease including ulcerative colitis, autoimmune disease including crohns disease and multiple sclerosis as well as other chronic inflammatory conditions, by administration of autologous or allogeneic cell suspension comprising MSCs the treating physician makes the assessment of an appropriate time at which to administer a therapeutic dose on the basis of various factors, such as pain, neurological function, mobility, endosopic examination, histologic examination of biopsies and imaging. However, each of these methods has disadvantages and in many cases significant tissue damage occurs before symptoms change in an appreciable way. In some cases the assessment tools are invasive, such as endoscopy and histological examination of biopsies. In other cases the patient may not be able to provide an accurate assessment of symptoms to guide the treating physician. Biomarkers are able to provide a powerful adjunct to imaging and other assessment tools, by quantifying the extent of damage at the molecular level. The inventor anticipates that biomarker analysis enables early stage diagnosis of disease status before imaging and other assessment techniques will be able to accurately reflect that.

In known methods for the treatment of conditions characterised by cartilage damage or degeneration, such as OA, RA as well as other chronic conditions and acute cartilage injuries, by administration of autologous or allogeneic adipose tissue-derived cell suspension the treating physician makes the assessment of an appropriate time at which to administer a therapeutic dose on the basis of various factors, such as pain and mobility and imaging. However, each of these methods has disadvantages. For example, assessment based on the physical condition of the affected joint, such as by imaging by magnetic resonance imaging (MRI) is expensive and potentially time-consuming in the situation where sufficient expertise and or infrastructure is not readily available. Where the assessment is based on subjective measures, such as self-reporting of pain scores or mobility, difficulties may arise. For example, self-reported pain and quality of life scores do not always correlate well with actual tissue damage, ie. in OA they may not correlate well with the extent of cartilage damage. There are many ways to image joints, including X-ray and MRI, however each of these is imperfect at providing a very detailed view of the extent of tissue damage. Biomarkers are able to provide a powerful adjunct to imaging and pain scores, by quantifying the extent of damage at the molecular level. The inventors anticipate that biomarker analysis enables early stage diagnosis of disease status before imaging techniques will be able to accurately reflect that.

Additionally, one of the issues with pain and mobility is that by the time the patient is reporting these systems the disease has progressed and further damage in the joint has occurred. By treating prior to the onset of symptoms the progression of the disease can be halted or slowed. The same holds true for other inflammatory conditions.

Implicit in the latter form of assessment is that the patient's condition will have deteriorated, or at least their discomfort levels will have increased and or their mobility has further deteriorated, before a decision to administer a further dose has been made. As an alternative to any form of assessment specific to the individual patient, a course of treatment may be based on periodical administration, such as a further dose being administered after a certain period of time has elapsed since the previous dose, for example, three months after, or six months after. A disadvantage of a simple time-based assessment is that it may have no specific relevance to the individual's particular circumstances, including that the level of degradation or inflammation will be dependent on many things, such as weight, exercise level and type of exercise.

The inventor has identified that MIF is detectable in serum from patients who have an inflammatory condition and that the detactable levels can act as a marker for clinical progression of the inflammation or condition. Thus as the inflammatory condition deteriorates the detectable levels of MIF increase, whereas as the condition improves the detectable levels decrease. As a consequence there is described herein a method for monitoring disease progression in a patient having an inflammatory condition, the method comprising determining the level of a biomarker MIF in at least a first and a second biological sample from said patient, wherein a change in the level of said biomarker in said second compared to said first biological sample is indicative of disease progression.

This capability to monitor disease progression also finds usefulness in assisting a clinician to improve or optimise a treatment regimen for a patient having an inflammatory condition. For example the invention provides a method of treating an inflammatory condition in a subject requiring said treatment, the method comprising administering to said subject a cell suspension comprising mesenchymal stem cells (MSCs), wherein the method further comprises determining the level of a biomarker MIF in at least a first and a second biological sample from said patient.

The inventor has also identified that cartilage specific collagen fragment (C-telopeptide of type II collagen; CTX-II) is detectable in the urine in serum, that COMP is detectable in serum, and that macrophage migration inhibitory factor (MIF) is detectable in serum from patients being treated for OA, and other musculoskeletal conditions. In particular, as described herein CTX-II levels decreased in the treatment group but increased significantly in the placebo group between baseline (ie. prior to treatment) and 6 months. A significant reduction in the serum level of MIF, a key cytokine involved in cartilage degradation pathway, was observed in the treatment group. The inventor thus describes herein that the detection of one or more of COMP, CTX-II and MIF in biological samples from the patient may be used to assist the treating physician to determine an appropriate or an optimal time at which to administer a dose of the autologous adipose tissue-derived cell suspension. As these biomarkers are postulated by the inventors to be indicative of changes in the pathology of the cartilage being treated, the methods disclosed herein also have application to treatment of other conditions characterised by cartilage damage or degeneration, such as RA as well as other chronic conditions and acute cartilage injuries.

Although the Examples herein demonstrate that the methods of the invention are appropriate when mesenchymal stem cells such as from culture expanded allogeneic umbilical cord blood derived MSCs or when autologous cell suspensions are the therapeutic agent, PCT/AU2009/001070 (WO2010/020005) entitled "Therapeutic methods using adipose tissue derived cell suspension comprising adipocytes", the contents of which are incorporated herein by reference, demonstrates that allogeneic cell suspensions are also used to treat such conditions. Accordingly, the methods of the invention herein also have application in assisting in the appropriate treatment of patients using allogeneic adipose tissue-derived cell suspensions.

The first and second biological samples may be any two samples from the patient separated in time of collection. The reference to "first" and "second" is not intended to indicate that they are chronologically the first and the second samples collected from the patient; they are simply two samples which have been obtained from the patient at different times, the "second" having been obtained after the "first" sample. It will be understood therefore that additional samples may have been obtained from the patient at any time before, or after, or between the samples referred to as the "first" and "second" samples in which the levels of the biomarker is determined. Any or all of the additional samples may also be analysed for biomarker levels.

It will also be understood that reference to determining the level of a biomarker in, for example, a first biological sample may mean determining the level in two samples taken at the same time point, for example where one of the biomarkers requires or is desired to be determined in serum and the other biological marker being determined at the same time point sample is required or desired to be determined in a urine sample. To further illustrate the context, the method may comprise determining the level of MIF and of CTX-II in, for example, a first biological sample. If the level of MIF is to be determined using a serum sample and the level of CTX-II is to be determined using a urine sample, the serum and the urine samples are obtained at the same time and so, in this context, they represent collectively a first biological sample. Hence a biological sample may consist of two samples of different type (eg. one urine and one serum) obtained from the patient at the same time. Although illustrated and explained in terms of a first biological sample, the same applies equally to any other numerical sample.

The first biological sample may be a sample obtained from the patient prior to commencement of the treatment. Such a sample may also be referred to as a baseline sample. In practice this sample may be obtained on the same day as the commencement of the treatment and in practice may be obtained soon after administration of the first dose of treatment as it would be expected that the detectable levels of the biomarker would not immediately be affected by administration of a first dose of the treatment. Hence it will be understood that reference to a first or baseline biological sample obtained from a patient "prior to" commencement of the treatment also encompasses a biological sample obtained from said patient within about 24 hours after administration of an initial dose of the treatment, for a biomarker that is known not to change from detectable pre-treatment levels within about 24 hours from a treatment dose.

The first biological sample may be a sample from the patient after commencement of treatment of the inflammatory condition. In an illustrative embodiment, for example, the first biological sample may be a sample from the patient approximately one month after commencement of the treatment and the second biological sample may be a sample from the patient after approximately three, four, five, or six months after commencement of the treatment. In this scenario, an improvement in a patient's condition as a result of a therapeutic dose may be reflected in a decreased level of MIF in the first post-treatment sample compared to the pre-treatment or baseline levels, and an increase in a second post-treatment level of MIF compared to the first post-treatment sample may indicate a deterioration in the underlying condition. In this manner the physician may employ the methods of the invention to monitor for regression or deterioration of the inflammatory condition, for example after an initial improvement or stabilisation of the condition. As a consequence, the treating physician may determine that it is appropriate to administer a further dose of the MSCs, or the autologous adipose tissue-derived cell suspension comprising adipose tissue-derived non-adipocyte cells, or the allogeneic adipose tissue-derived cell suspension comprising adipose tissue-derived non-adipocyte cells. Typically, the further dose would be a portion of the autologous adipose tissue-derived cell suspension comprising adipose tissue-derived non-adipocyte cells that had been administered to the patient at the commencement of the treatment, the portion having been stored frozen prior to use.

In this manner the method provides the physician with an objective measure by which to characterise the progression of the inflammatory condition in the individual patient. The method may be used in conjunction with other measures available to the physician for assessing the state of the inflammatory condition of the patient to determine an appropriate time to administer a second or subsequent dose of the MSCs or cell suspension, such as an increase or decrease in the mobility of the patient or the affected joint, an increase or decrease in the pain scores reported by the patient, or analysis by ultrasound or MRI, changes in the cognitive ability of the patient, or changes in the abdominal discomfort of the patient. The specific additional criteria may depend on the underlying nature of the inflammatory condition.

In an illustrative embodiment, for example, the first biological sample may be a sample from the patient approximately one month after commencement of the treatment and the second biological sample may be a sample from the patient after approximately three, four, five, or six months after commencement of the treatment. In this scenario, an improvement in a patient's condition as a result of a dose may be reflected in a decreased level of one or more of biomarkers COMP, CTX-II and MIF in the first post-treatment sample compared to the pre-treatment or baseline levels, and an increase in a second post-treatment level of one or more of biomarkers COMP, CTX-II and MIF compared to the first post-treatment sample may indicate a deterioration in the underlying condition. In this manner the physician may employ the methods of the invention to monitor for regression or deterioration of the condition, for example after an initial improvement or stabilisation of the condition. As a consequence, the treating physician may determine that it is appropriate to administer a further dose of the autologous adipose tissue-derived cell suspension comprising adipose tissue-derived non-adipocyte cells. Typically, the further dose would be a portion of the autologous adipose tissue-derived cell suspension comprising adipose tissue-derived non-adipocyte cells that had been administered to the patient at the commencement of the treatment, the portion having been stored frozen prior to use.

In this manner the method provides the physician with an objective measure by which to characterise the progression of the condition in the individual patient, such as the OA, RA, or cartilage injury. The method may be used in conjunction with other measures available to the physician for assessing an appropriate time to administer a second or subsequent dose of the cell suspension, such as an increase or decrease in the mobility of the patient or the affected joint, an increase or decrease in the pain scores reported by the patient, or analysis by ultrasound or MRI.

By monitoring the level of MIF and optionally of one or more of the biomarkers COMP and CTX-II the physician may be able to identify deterioration in, for example, the affected joint before the patient experiences or reports an increase in discomfort or pain scores for the joint or before they experience or report a decrease in mobility of the affected joint. By administering a second or subsequent dose on the basis of the methods described herein, the physician may be able to reduce the effect or longevity of such deterioration, such as by reducing what might otherwise have been a significant increase in pain experienced by the patient or a significant decrease in mobility experienced by the patient.

Monitoring the level of MIF and optionally of one or more of the biomarkers COMP and CTX-II may comprise the accumulation over time of a patient-specific timeline of the levels of MIF and optionally at least one of COMP and CTX-II in samples from the patient. For example, the physician may have a "baseline" assessment comprising one or more samples from the patient prior to commencement of the treatment and may have any number of samples from the patient after commencement of the treatment. The samples may be obtained at regular intervals, such as monthly, or three monthly or six monthly, or they may be obtained at irregular intervals, such as at any period separated by one week to six or twelve months. Where relatively rapid changes in the underlying condition or in the level of one or more of the biomarkers is expected, the time interval between samples would typically be short in order to allow the physician to closely monitor the patient for relevant changes. Where relatively slow changes in the underlying condition or in the level of one or more of the biomarkers is expected, samples would typically be obtained less frequently.

Determining the level of MIF and optionally at least one biomarker selected from COMP and CTX-II in the biological sample or samples may be done simultaneously, such that the first, second and, if present, additional samples may all be determined at the same time. Alternatively, one or more of the sample(s) may be assessed for the biomarker or biomarkers at a different time to the other sample or samples. For example, each sample may be assessed for the level of biomarker soon after collection.

Drawing on the examples herein, embodiments of the invention may be illustrated as follows.

As shown herein MIF and optionally the biomarkers COMP and CTX II may be used as indicators of retreatment. There will be natural biological variation between patients, but successful adipose tissue -derived cell suspension treatment will decrease MIF and stabilise or decrease CTX II and stabilise or decrease COMP. Post-treatment monitoring will indicate when the cartilage degradation process has reached problematic levels, with the pre-treatment baseline levels of COMP, CTX II and MIF being key points. When post-treatment levels of COMP, CTX and MIF increase, for example either near to or back to pre-treatment levels, the physician would have a strong indication that cartilage degradation is active.

The invention thus includes the use of multiple biomarkers that when used in combination, provide indications about the status of cartilage degradation and hence of conditions characterised by cartilage degradation, such as OA, RA and cartilage injuries. Further included in the invention is the use of the individual biomarker MIF, to provide an indication of the status of an inflammatory condition prior to and during treatment with mesenchymal stem cells, including with autologous or allogeneic adipose tissue-derived cell suspensions.

Methods of treating osteoarthritis and other conditions referred to herein

As described in co-pending Australian patent application no. 2013204930, entitled "Therapeutics using multiple injections of cells", the contents of which are incorporated herein by reference, a course of treatment of osteoarthritis using multiple administration of an autologous adipose tissue-derived cell suspension comprising adipose tissue-derived non-adipocyte cells administered over a period of time to the subject can provide improved outcomes for the subject, compared to previously known treatments. In this manner, the autologous adipose tissue-derived cell suspension comprising adipose tissue-derived non-adipocyte cells is typically one which has been prepared from a single adipose tissue extraction from the patient, divided into useful portions or aliquots, a first dose being administered soon after preparation of the cell suspension and other portions being stored under appropriate conditions until required for the second and, where appropriate, third, fourth, fifth, etc doses at which time the required dose is retrieved from storage and administered to the subject.

As shown in for example PCT/AU2009/001070 (WO2010/020005) entitled "Therapeutic methods using adipose tissue derived cell suspension comprising adipocytes", the contents of which are incorporated herein by reference, allogeneic cell suspensions may also be used for the treatment of such conditions and so the methods described herein apply also to treatments utilising allogeneic adipose tissue-derived cell suspensions.

As shown in, for example, PCT/AU2012/001140 (WO2013/040649) entitled Therapeutics using adipose cells and cell secretions", the contents of which are herein incorporated by reference, compositions comprising a combination of MSCs, such as of an adipose tissue-derived non-adipocyte cells, and of cell secretions, may also be used for the treatment of such conditions mentioned herein and so the methods described herein apply also to treatments using such combinations.

The instant invention provides further improvements to methods of treating osteoarthritis and other conditions involving cartilage degeneration, damage or trauma, by providing methods for monitoring clinical progression in a patient having OA or the condition, which methods can be used independently of the methods for treatment of OA or the condition, or may preferably be used in conjunction with methods for such treatment. The methods herein also apply to the treatment of inflammatory conditions.

In particular, the methods of the present invention may provide benefit to the treating physician or to the patient when integrated into the overall treatment plan for the patient, such as by utilising a combined method in which the patient is treated with a course of treatment comprising multiple doses over time of an autologous adipose tissue-derived cell suspension comprising adipose tissue-derived non-adipocyte cells, wherein a first dose comprises a portion of a freshly prepared cell suspension and a subsequent dose or doses comprise a portion of the cell suspension that has been stored frozen, and in which the method also comprises determining the level of MIF and optionally at least one biomarker selected from COMP and CTX-II in at least a first and a second biological sample from said patient. In this treatment course the timing of administration of one or more of the subsequent doses is determined on the basis of the level of the at least one biomarker, for example where the level of the at least one biomarker is greater in the second compared to the first biological sample.

The level of at least the biomarker MIF is determined, either solely or additionally with one or more of COMP and CTX-II.

Inflammatory disorders

The methods described herein are applicable to treatment of an inflammatory disorder and/or for alleviating pain associated with an inflammatory disorder in a subject. It will be understood that the term inflammatory disorder and inflammatory condition may be used interchangeably herein, unless the context indicates otherwise.

Inflammation may arise as a response to an injury or abnormal stimulation caused by a physical, chemical, or biologic agent. An inflammation reaction may include the local reactions and resulting morphologic changes, destruction or removal of the injurious material, and responses that lead to repair and healing. The term "inflammatory" when used in reference to a disorder refers to a pathological process which is caused by, resulting from, or resulting in inflammation that is inappropriate or which does not resolve in the normal manner. Inflammatory disorders may be systemic or localized to particular tissues or organs.

Inflammation is known to occur in many disorders which include, but are not limited to: Systemic Inflammatory Response (SIRS); Alzheimer's Disease (and associated conditions and symptoms including: chronic neuroinflammation, glial activation; increased microglia; neuritic plaque formation; and response to therapy); Amyotropic Lateral Sclerosis (ALS), arthritis (and associated conditions and symptoms including, but not limited to: acute joint inflammation, antigen-induced arthritis, arthritis associated with chronic lymphocytic thyroiditis, collagen-induced arthritis, juvenile arthritis; rheumatoid arthritis, osteoarthritis, prognosis and streptococcus-induced arthritis, spondyloarthropathies, gouty arthritis), asthma (and associated conditions and symptoms, including: bronchial asthma; chronic obstructive airway disease; chronic obstructive pulmonary disease, juvenile asthma and occupational asthma); cardiovascular diseases (and associated conditions and symptoms, including atherosclerosis; autoimmune myocarditis, chronic cardiac hypoxia, congestive heart failure, coronary artery disease, cardiomyopathy and cardiac cell dysfunction, including: aortic smooth muscle cell activation; cardiac cell apoptosis; and immunomodulation of cardiac cell function; diabetes and associated conditions, including autoimmune diabetes, insulin-dependent (Type 1) diabetes, diabetic periodontitis, diabetic retinopathy, and diabetic nephropathy); gastrointestinal inflammations (and related conditions and symptoms, including celiac disease, associated osteopenia, chronic colitis, Crohn's disease, inflammatory bowel disease and ulcerative colitis); gastric ulcers; hepatic inflammations such as viral and other types of hepatitis, cholesterol gallstones and hepatic fibrosis, HIV infection and associated conditions, including degenerative responses, neurodegenerative responses, and HIV associated Hodgkin's Disease, Kawasaki's Syndrome and associated diseases and conditions, including mucocutaneous lymph node syndrome, cervical lymphadenopathy, coronary artery lesions, edema, fever, increased leukocytes, mild anemia, skin peeling, rash, conjunctiva redness, thrombocytosis; inflammatory disorders of the skin, including dermatitis, such as atopic dermatitis and associated conditions; multiple sclerosis, nephropathies and associated diseases and conditions, including diabetic nephropathy, endstage renal disease, acute and chronic glomerulonephritis, acute and chronic interstitial nephritis, lupus nephritis, Goodpasture's syndrome, hemodialysis survival and renal ischemic reperfusion injury, neurodegenerative diseases and associated diseases and conditions, including acute neurodegeneration, induction of IL-I in aging and neurodegenerative disease, IL-I induced plasticity of hypothalamic neurons and chronic stress hyperresponsiveness, ophthalmopathies and associated diseases and conditions, including diabetic retinopathy, Graves' ophthalmopathy, and uveitis, osteoporosis and associated diseases and conditions, including alveolar, femoral, radial, vertebral or wrist bone loss or fracture incidence, postmenopausal bone loss, mass, fracture incidence or rate of bone loss, otitis media (adult or paediatric), pancreatitis or pancreatic acinitis, periodontal disease and associated diseases and conditions, including adult, early onset and diabetic; pulmonary diseases, including chronic lung disease, chronic sinusitis, hyaline membrane disease, hypoxia and pulmonary disease in SIDS; restenosis of coronary or other vascular grafts; rheumatism including rheumatoid arthritis, rheumatic Aschoff bodies, rheumatic diseases and rheumatic myocarditis; thyroiditis including chronic lymphocytic thyroiditis; urinary tract infections including chronic prostatitis, chronic pelvic pain syndrome and urolithiasis, immunological disorders, including autoimmune diseases, such as alopecia aerata, autoimmune myocarditis, Graves' disease, Graves ophthalmopathy, lichen sclerosis, multiple sclerosis, psoriasis, systemic lupus erythematosus, systemic sclerosis, thyroid diseases (e.g. goitre and struma lymphomatosa (Hashimoto's thyroiditis, lymphadenoid goitre), sleep disorders and chronic fatigue syndrome and obesity (non-diabetic or associated with diabetes), resistance to infectious diseases, such as Leishmaniasis, Leprosy, Lyme Disease, Lyme Carditis, malaria, cerebral malaria, meningitis, tubulointerstitial nephritis associated with malaria), which are caused by bacteria, viruses (e.g. cytomegalovirus, encephalitis, Epstein-Barr Virus, Human Immunodeficiency Virus, Influenza Virus) or protozoans (e.g., Plasmodium falciparum, trypanosomes), response to trauma, including cerebral trauma (including strokes and ischemias, encephalitis, encephalopathies, epilepsy, perinatal brain injury, prolonged febrile seizures, SIDS and subarachnoid hemorrhage), low birth weight (e.g. cerebral palsy), lung injury (acute hemorrhagic lung injury, Goodpasture's syndrome, acute ischemic reperfusion), myocardial dysfunction, caused by occupational and environmental pollutants (e.g. susceptibility to toxic oil syndrome silicosis), radiation trauma, and efficiency of wound healing responses (e.g. burn or thermal wounds, chronic wounds, surgical wounds and spinal cord injuries), septicemia, hypothyroidism, oxygen dependence, cranial abnormality, early onset menopause, a subject's response to transplant (rejection or acceptance), acute phase response (e.g. febrile response), general inflammatory response, acute respiratory distress response, acute systemic inflammatory response, wound healing, adhesion, immunoinflammatory response, neuroendocrine response, fever development and resistance, acute-phase response, stress response, disease susceptibility, repetitive motion stress, tennis elbow, ligament and tendon problems, and pain management and response.

In particular embodiments the inflammatory disorder is a joint-related inflammatory disorder, such as arthritis.

The methods and compositions of the invention may be used for the treatment of ligament injuries and tendon injuries or for the alleviation of pain associated with such injuries. Ligament injuries and tendon injuries, in some forms, can be classified as inflammatory disorders. Some ligament injuries and tendon injuries may not be considered inflammatory disorders. For the avoidance of doubt, ligament injuries and tendon injuries contemplated in this invention may be those which are inflammatory disorders or are associated therewith and those which may not be considered inflammatory disorders.

The following paragraphs describe source and preparation of the adipose tissue-derived cell suspensions, cell suspensions comprising MSCs, and methods by which a course of such treatment may be administered to a patient.

Adipose tissue

The cell suspensions used in the treatment methods of the invention are autologous adipose tissue-derived cell suspensions or allogeneic adipose tissue-derived cell suspensions. Adipose tissue may be human adipose tissue or mammalian animal adipose tissue, depending on the subject of the treatment and depending on whether autologous or allogeneic cell suspension is to be used. The adipose tissue may comprise "white" adipose tissue, or "brown" adipose tissue.

The adipose tissue may originate from any source in the subject's body, or in the case of allogeneic material the donor's body, which is accessible. Subcutaneous fat, for example, is readily accessible with only superficial wounding, or by using "keyhole surgery" techniques. For example adipose tissue may be tissue collected using liposuction techniques, or adipose tissue which is removed with reproductive tissue when de-sexing a male or female animal. The adipose tissue may be collected during a cosmetic procedure performed on the subject of the intended treatment. The adipose tissue may be collected specifically as part of the intended treatment of the subject for the indicated condition. The adipose tissue may be rinsed with a tissue culture medium or buffered isotonic solution to remove adherent blood cells, and may be trimmed or coarsely processed to remove large blood vessels or connective tissue elements prior to generating an adipose tissue-derived cell suspension.

The adipose tissue may be derived from a mature animal or from a juvenile animal.

In particular embodiments the subject or patient is a companion animal, such as a canine or a feline domestic animal, or a working animal. In other particular embodiments the subject or patient is a farm animal or racing animal selected from a horse, donkey, ass, cow, buffalo, sheep, goat, camel or pig. In other particular embodiments the subject or patient is a human. Typically, where the patient is a human, the adipose tissue is autologous.

Adipose Tissue-Derived Cell Suspension

The term "adipose tissue-derived cell suspension" as used herein encompasses isolated cells from adipose tissue or small aggregates or pieces of adipose tissue, or a mixture of two or more of: isolated cells, small aggregates and pieces of adipose tissue. The adipose tissue-derived cell suspension comprises adipose tissue-derived non-adipocyte cells. The cell suspension may be obtained by mechanically dissociating adipose tissue using techniques which are readily available in the art. Any suitable method for the mechanical dissociation of adipose tissue may be used, for example by mincing adipose tissue with blades, or with scissors, or by forcing adipose tissue through screens or meshes with a pore size sufficient to break the tissue into isolated cells or small pieces of adipose tissue, or a combination of these techniques. Small aggregates of adipose tissue may form when dissociated adipose-derived cells reassociate into larger assemblies, for example on standing in a medium. Small pieces or aggregates of adipose tissue may be less than ten millimetres in diameter, less than five millimetres in diameter, less than one millimetre in diameter,

less than 500 $\mu$m in diameter or less than 250 $\mu$m in diameter.

The adipose tissue-derived cell suspension may be filtered through a mesh or screen to remove cell aggregates or tissue pieces which are greater than the mesh or screen pore size.

Proteolytic enzymes may be used to promote the dissociation of adipose tissue into an adipose tissue-derived cell suspension. Enzymes which are suitable for such a use are well known in the art, and include but are not limited to trypsin, and collagenase. It is usual to remove and/or otherwise inactivate the proteolytic enzymes before using the adipose-tissue-derived cell extract, as these enzymes may not be compatible with a desired in vivo use of the cells. The proteolytic enzymes may be used in combination with techniques for the mechanical dissociation of adipose tissue to generate an adipose tissue-derived cell suspension.

A mechanical dissociation technique may be used without using one or more proteolytic enzymes. The technique used in this manner may be used to rapidly generate an adipose tissue-derived cell suspension.

The cell suspension may be suspended in a liquid. The liquid may be added to the adipose tissue before, during or after the dissociation of the adipose tissue. The liquid may comprise a medium which is capable of maintaining adipose tissue cell survival for at least 24 hours under appropriate culture conditions. The liquid may comprise an isotonic buffered solution, such as a phosphate or a HEPES buffered saline, which is capable of maintaining adipose tissue cell survival for at least one hour. The liquid may comprise a tissue culture medium. The liquid may comprise serum or serum components which support or extend adipose tissue cell survival in the cell suspension. The serum or serum components may be autologous serum or serum components.

In some embodiments the cell suspension may not have added liquid, but instead the cells are suspended in liquid which is formed during the dissociation of the tissue.

The preparation of an adipose tissue-derived cell suspension may comprise a centrifugation step. The centrifugation of isolated cells or small aggregates or pieces of adipose tissue suspended in a liquid, such as a medium, is at approximately 500 g for 10 minutes, or for sufficient time and at a sufficient g-force to generate a cell pellet which comprises adipose-derived non-adipocyte cells, above which is a layer of medium, floating above which in turn is a layer which comprises the viable adipocytes, and floating at the top is a layer of lipid which is derived from ruptured adipocytes. Following centrifugation, in certain embodiments the lipid layer and the medium layer will be discarded and the retained cells are mixed, leaving an adipose tissue-derived cell suspension which comprises viable adipocytes and adipose-derived non-adipocyte cells. In other embodiments, only the layer comprising the viable adipocytes will be retained.

In other embodiments, the layer comprising adipocytes may be removed and hence not included in the adipose tissue-derived cell suspension. This will typically occur when preparing an adipose tissue-derived cell suspension which is substantially free of adipocytes. A cell suspension referred to herein as being substantially free of adipocytes means that the cell suspension has been significantly depleted of adipocytes compared to the starting material, such as by removal of the adipocyte fraction after centrifugation. It will be understood that substantially free of adipocytes when used in relation to a cell suspension includes complete absence of adipocytes and also includes the situation where minimal retention of adipocytes in the material has occurred.

In other embodiments, only part of the adipocyte content of the adipose tissue may be removed in the preparation of the adipose tissue-derived cell suspension. In this case, the resultant cell suspension will comprise adipocytes, but at a reduced proportion relative to other retained components, such as the stem cells, compared to the proportion in the starting material. In an embodiment the adipose tissue-derived cell suspension comprises at least 10% adipocytes by volume. In an embodiment the adipose tissue-derived cell suspension comprises between 10% and 30% adipocytes by volume. In an embodiment the adipose tissue-derived cell suspension comprises at least 10% adipocytes by number (that is, at least 10% of the total number of cells in the cell suspension are adipocytes). In an embodiment the adipose tissue-derived cell suspension comprises at least 20% adipocytes by number. In an embodiment the adipose tissue-derived cell suspension comprises at least 30% adipocytes by number. In an embodiment the adipose tissue-derived cell suspension comprises between 10% and 30% adipocytes by number.

One centrifugation step or multiple centrifugation steps may be used, for example to provide additional cell separation steps. In other embodiments, the preparation of an adipose tissue-derived cell suspension does not include a centrifugation step.

The adipose tissue-derived cell suspension may or may not comprise viable adipocytes. When present, the adipocytes may retain detectable quantities of lipid in their cytoplasm, and may be separated from adipose-derived non-adipocyte cells on the basis of the different density provided by the lipid. Lipid may be detectable using light microscopy techniques, including phase contrast microscopy, or by staining a sample of cells with a lipophilic dye such as Oil Red O. Adipocytes which retain lipid in their cytoplasm are considerably more fragile than other adipose-derived cells, and accordingly where viable adipocytes are desired techniques for dissociating tissue which damage or kill a large proportion of the adipocytes should be avoided. The ultrasonic dissociation of adipose tissue or techniques in which adipose tissue is vigorously shaken, for example, are unlikely to provide a cell suspension which contains large numbers of viable adipocytes. The viability of adipocytes may readily be determined using readily available techniques, such as the LIVE/DEAD cell viability assays (Life Technologies).

The adipose tissue-derived cell suspension may comprise both adipocytes and adipose-derived non-adipocyte cells. The adipose-derived non-adipocyte cells typically include cells of the stromal vascular fraction, including mesenchymal stem cells. Cells of the stromal vascular fraction typically pellet upon centrifugation conditions described herein of an adipose tissue-derived cell suspension.

In embodiments which comprise both adipocytes and adipose-derived non-adipocyte cells, the adipose tissue-derived cell suspension may be conveniently prepared by methods which comprise a centrifugation step, as described herein, in which both the adipocyte cell layer and the pelleted adipose-derived non-adipocyte cells are collected. Alternatively, in these embodiments the adipose tissue-derived cell suspension may be prepared by dissociating adipose tissue as described herein without a centrifugation step.

The adipose tissue-derived cell suspension or a portion thereof, optionally comprising adipocytes, may be stored under appropriate conditions. The storage conditions typically permit the retention of cell viability of some or all cells in the cell suspension, such as greater than 50%, greater than 60%, greater than 70%, greater than 80%, greater than 90%, or greater than 95%.

Where the adipose tissue-derived cell suspension or a portion thereof is to be stored frozen it may be in any carrier liquid appropriate for freezing of cells. As an illustrative but not limiting example, the cells may be suspended in culture medium, which may be serum-containing or serum-free, such as DMEM, RPMI, minimal essential media, or in serum prior to freezing.

The adipose tissue-derived cell suspension typically also comprises autologous serum or plasma, which may be added during the preparation of the cell suspension or at a late stage of the preparation, such as when the cell pellet is separated from components not desired in the cell suspension being prepared.

Where the adipose tissue-derived cell suspension is to be stored frozen, the cells suspension may be combined with a composition comprising cell secretions. The composition comprising cell secretions may comprise, for example clarified media from culture of an adipose tissue-derived cell suspension or may comprise concentrated media from culture of an adipose tissue-derived cell suspension. Such methods for storage of cells, cell suspension and in particular adipose tissue-derived cell suspensions are disclosed in PCT/AU2012/001140 (WO2013/040649) entitled "Therapeutics using adipose cells and cell secretions", the contents of which are incorporated herein by reference.

Where the adipose tissue-derived cell suspension is to be stored frozen it typically also comprises a cryopreservative. It will be understood that any additives and method for storing the cell suspension without significant loss of cell viability over time may be used. For example, methods for the storage of mesenchymal stem cells are known in the art. As an example, the cell suspension may comprise dimethylsulfoxide (DMSO) or glycerol, at an appropriate concentration, such as 5% to 10%. As further examples, the cell suspension may comprise one or more cryopreservative sugars, such as trehalose, dextran, dextrose, sucrose at an appropriate concentration. For example, a cryopreservative sugar may be included at a concentration in the range of 1% w/v to 30% w/v. In a further example, a cryopreservative sugar may be included at a concentration in the range of 5% w/v to 10% w/v.

The constituents of the cell suspension, such as the liquid medium and the cryopreservative, are typically pharmaceutically acceptable at the concentrations used. This has the advantage that the adipose tissue-derived cell suspension can be administered to the subject after thawing with minimal post-thaw processing.

The cell suspension is intended as part of a course of treatment for the condition afflicting the subject. In that manner where autologous cell suspension is used the course comprises multiple doses of the cell suspension over a period of time to the subject from which the adipose tissue was obtained and from which the cell suspension was prepared. The time course of the treatment will typically be over weeks, to months and potentially a year or more. For ease of use the adipose tissue-derived cells suspension is typically divided into useful portions or aliquots soon after preparation and the material to be used for the second and, where appropriate, third, fourth, fifth, etc doses is then stored frozen for retrieval at an appropriate time. Typically, the stored material will be in portions or aliquots which comprise a single dose.

Typically, where the treatment utilizes autologous cell suspension, at least one aliquot or portion of the cell suspension for administration as a first dose of the course of treatment will be obtained from the prepared cell suspension prior to addition of the cryopreservative or other components intended to assist in the storage of the cell suspension.

The cell suspension may be referred to as a pharmaceutical composition as it typically also comprises one or more of a pharmaceutically acceptable carrier diluent, excipient or adjuvant.

The cell suspension is typically frozen under controlled conditions to minimize cell damage, for example by slow freezing, typically at a rate of about 1°C/min, such as by placing in a programmable freezing device, or in an insulated container in a -70°C to -90°C freezer. For storage, frozen cells are typically then transferred to liquid nitrogen storage.

Where the course of treatment utilizes allogeneic adipose tissue-derived cell suspension, all doses may be stored frozen prior to use. In such a course of treatment one or more doses may originate from different donors.

A cell processing method and device which may be used for the preparation of adipose tissue-derived cell suspensions is described in co-pending application PCT/AU2012/000272 (WO2012/122603) entitled "Cell processing method and device", the contents of which are incorporated herein by reference.

Isolation of MSCs

Mesenchymal stem cells (MSCs) may be used in any of the methods of the invention. MSCs can be obtained from any tissue in the body. Sources include bone marrow, adipose tissue and umbilical cord blood. Bone marrow (BM) contains both MSCs and Hematopoeitc Stem Cells (HSCs). In BM, MSCs are present at lower numbers than HSCs at 10 and 100 cells in every million BM cells respectively. BM is harvested using an aspiration needle such as a jamshidi. A 10 mL volume of BM will contain approximately $6 \times 10^7$ nucleated cells, of which 600 to 6000 are stem cells. To separate the nucleated cells from red blood cells, a density gradient centrifugation procedure such as Ficoll-paque is performed. After washing by gentle centrifugation in saline, cells can be administered immediately, or can be further purified or enriched by tissue culture and or immunological separation methods. In embodiments, the MSCs may be allogeneic or may be autologous. In embodiments, the MSCs may be culture expanded MSCs.

MSCs are present in fat at higher levels than bone marrow. The most likely explanation for this finding is that MSCs, along with other nucleated cell types, are associated with the vast network of dense capillary beds lining adipose tissue. Adipose tissue can be harvested by surgical excision or by liposuction, and methods for preparing non-adipocyte cell suspensions, including suspensions comprising MSCs, from adipose tissue are described herein.

In our experience, the number of MSCs recovered from adipose tissue ranges from 50,000 to 5 million per gram.

Umbilical cord blood is the blood that remains in the vein of the umbilical cord and placenta at the time of birth. This blood is rich in hematopoietic stem cells and also contains low numbers of MSCs. Higher numbers of MSCs are present in the Wharton's jelly which is the tissue surrounding the umbilical vein and vessels in the cord.

MSCs can also be isolated from other sources, such as peripheral blood, synovium, muscle, periosteum, dental pulp, placenta.

Methods of treatment

In embodiments of methods of the invention a course of treatment is provided to a subject having a condition characterized by cartilage damage or degeneration, such as osteoarthritis, rheumatoid arthritis or a cartilage injury, in which a first dose of the autologous adipose tissue-derived cell suspension comprising adipose tissue-derived non-adipocyte cells is administered to the subject soon after preparation of the cell suspension. In this context the first dose may be described as being administered to the subject as a freshly prepared cell suspension. In this context the term freshly prepared and similar terms used herein means that the cell suspension so administered is administered to the subject on the same day as it is prepared. As described herein the adipose tissue-derived cell suspension is prepared from adipose tissue obtained from the subject to whom the course of treatment is to be administered, hence the resultant cell suspension is an autologous cell suspension. Typically, the time taken from isolation of the adipose tissue from the subject through to the prepared cell suspension ready for administration is up to about two to three hours. A sample freshly prepared is therefore one which is ready for administration to the subject within about two to three hours of removal of the adipose tissue from the subject.

As noted herein the methods of the invention, utilizing MIF and optionally the biomarkers COMP and CTX-II to assist in monitoring a patient's underlying condition, are also applicable to patients having a treatment using allogeneic adipose tissue-derived cell suspension.

As described herein any of the courses of treatment may use a cell suspension of mesenchymal stem cells. In a non-limiting example shown herein the umbilical MSC treatments for neurodegeneration and ulcerative colitis were allogeneic human treatment. Allogeneic culture expanded MSCs are described herein, for example for human treatment.

It will be understood that in the context of the methods of the invention a dose means the administration of the cell suspension to the subject at a given time, whether that dose be administered in a single application or in more than one application. As an illustrative example, a dose may consist of a single administration, such as a single injection into a targeted site on the subject's body. As a further illustrative example, a dose may consist of multiple administrations to one or more targeted sites on the subject's body, such as multiple injections. Any of the first, and or subsequent doses, such as any of the second, third, fourth, fifth, etc, doses may therefore be administered as a single application or as multiple applications.

The method may comprise a course of treatment comprising a first dose and a second dose, or a first dose, a second dose and a third dose, or a first dose, a second dose, a third dose and a fourth dose, or a first dose, second dose, a third dose, a fourth dose and a fifth dose, or any other number of doses that the medical practitioner considers appropriate to the patient.

In an embodiment the adipose tissue-derived cell suspension comprises aggregates of cells and or comprises pieces of adipose tissue. In an embodiment the adipose tissue-derived cell suspension further comprises adipocytes. In an embodiment the cell suspension is substantially free of adipocytes. In an embodiment the adipose tissue-derived cell suspension is prepared by a method that comprises removal of (i) part of the adipocyte content or (ii) substantially all of the adipocyte content during preparation of the adipose tissue-derived cell suspension.

In an embodiment the course of treatment comprises a first dose of a cell suspension comprising non-adipocyte cells and adipocytes and the cell suspension of one or more of the one or more subsequent dose or doses is substantially free of adipocytes. In an embodiment the course of treatment comprises a first dose of a cell suspension comprising non-adipocyte cells and adipocytes and the cell suspension of one or more of the one or more subsequent dose or doses comprises non-adipocyte cells and adipocytes.

In an embodiment the course of treatment comprises multiple doses administered over a total treatment period of between three and twelve months. In an embodiment the course of treatment comprises multiple doses administered over a total treatment period of between about three months and several years, such as one, two, three or more years. In an embodiment the course of treatment comprises multiple doses administered over a total treatment period of between six and twelve months. In an embodiment the course of treatment comprises multiple doses administered over a total treatment period of between three and nine months. In an embodiment the course of treatment comprises multiple doses administered over a total treatment period of between six and nine months.

In an embodiment the subsequent dose or doses is administered to the subject soon after thawing, such as within about 10 minutes after thawing, or within about 20 minutes after thawing, or within about 30 minutes after thawing or within about one hour of thawing or within about two hours of thawing.

In methods of the instant invention at least one of the doses, typically a second or subsequent dose, is administered to the patient after determination of the level of at least one biomarker selected from COMP, CTX-II and MIF in at least a first and a second biological sample from said patient. In an embodiment the level of at least two of the biomarkers COMP, CTX-II and MIF is determined in at least a first and a second biological sample from the patient. In an embodiment the level of the three biomarkers COMP, CTX-II and MIF is determined in at least a first and a second biological sample from the patient. In an embodiment substantially all of the doses is administered to the patient after determination of the level of at least one biomarker selected from COMP, CTX-II and MIF in at least a first and a second biological sample from said patient. In an embodiment the so-determined level of biomarkers indicates deterioration in the patient's condition, thereby providing an indication for the treating physician to administer a further dose to the patient.

An appropriate time period between the first and each subsequent dose may be different between different patients and even for a given patient, the intervals between doses may be variable. Using the methods of the invention allows the treating physician to administer a dose of the cell suspension to the patient on the basis of an objective measurement in a biomarker associated with disease progression. This provides the physician with an additional, improved basis upon which to determine an appropriate time for administration of a dose of the cell suspension. Prior to the instant invention, the timing of doses may be on the basis of a simple periodical application, such as every three months, in which case the patient may undergo more doses than is necessary, or the timing of doses may be on the basis of increased pain experienced by the patient, or decreased mobility experienced by the patient, which is undesirable. In this manner it is the intended course of treatment in the methods of the invention that the subject be administered multiple doses of the cell suspension over a period of time for the treatment of the same condition in the individual over that time.

In an embodiment the course of treatment comprises multiple doses in which each subsequent dose is separated in time from the previous dose by between one week and ten weeks. In an embodiment the course of treatment comprises multiple doses each subsequent dose separated in time from the previous dose by between two weeks and eight weeks. In an embodiment the course of treatment comprises multiple doses each subsequent dose separated in time from the previous dose by between two weeks and six weeks. For any given course of treatment the time period between each dose may or may not be a consistent period. As an illustrative example, the time period between the first and second dose may or may not be the same as the time period between the second and third dose.

The pharmaceutical composition may be administered to the subject patient at a site remote from the afflicted area. In this context, "remote" means that the administration is not direct application of the cell suspension to the site of inflammation or other injury or disease being treated where such a site is identifiable. As an illustration, in the case of treatment of an arthritic joint, administration as previously described in the art involved injection of adipose tissue-derived cell suspensions directly into the afflicted joint. Such administration requires a high degree of skill on the part of the treating physician or clinician to ensure appropriate precision. The handling of the affected limb or joint required in such administration also increases the distress experienced by the patient, be they human or non-human. By providing for the remote administration of adipose tissue-derived suspension the present invention offers improved methods, uses and compositions for the treatment of such diseases. For example, the remote administration may be by subcutaneous injection, such as in the scruff of the neck of an animal (for example a cat or dog) being treated, or by intramuscular injection. As a further example, administration to a dog by intramuscular injection may be in to thigh of the dog. As a further example, administration to a bovine by intramuscular injection may be in the caudal fold.

Biological sample

The biological sample is any suitable sample in which the desired biomarker may be detected. Typically, the sample is blood or a fraction thereof, such as plasma or serum. Methods are known in the art for collection of blood from a subject and for the separation of desired fractions of blood for testing purposes. In an embodiment the biological sample is synovial fluid. In an embodiment the biological sample is urine. In an embodiment the biomarker CTX-II is determined in a urine sample. In an embodiment the biomarker CTX-II is determined in a serum sample. In an embodiment the biomarker MIF is determined in a serum sample. In an embodiment the biomarker MIF is determined in blood or fluid such as synovial fluid. In an embodiment the further optional biomarker COMP is determined in a serum sample. In an embodiment the further optional biomarker COMP is determined in a urine sample.

The step of obtaining a biological sample from the patient may be undertaken as part of the methods of the invention or may be undertaken as a separate step. The step of obtaining a blood sample from an individual may be undertaken as part of a consecutive series of steps in the performance of the method of the invention. The step of obtaining a blood sample from an individual may be undertaken as a distinct step or steps separate from one or more remaining steps of the method of the invention, for example separate in time, location or operator. Accordingly, in the performance of the method of the invention obtaining the blood sample may or may not involve extraction of blood from said individual. Performance of the method of the invention may, for example, comprise receiving a blood sample in a container, the blood having previously been extracted from the individual as an exercise separate from the performance of the method of the invention. As a further example, obtaining a blood sample may comprise retrieving from temporary storage a blood sample extracted from the individual as an exercise separate from the performance of the method of the invention. It will be understood that the performance of the method of the invention may thus be conducted entirely *ex vivo*.

The sample may be tested to determine the presence or level of a biomarker soon after collection and, if desired, processing, or the sample, or a processed fraction thereof, maybe stored under appropriate conditions until testing. For example, the biological sample may be blood or a fraction thereof (serum or plasma preferably). For example, where the biological sample is urine thereof, it may be centrifuged first to remove debris and cells, and then stored prior to use for testing. These samples are ideally stored refrigerated for 1 or 2 hours and more preferably stored frozen.

Typically, where the patient is a human, the biological sample may be collected from a subject under the clinical care of a medical practitioner by, for example, a medical practitioner or a health care professional. A medical practitioner may be any person that is registered, authorized or certified under law to practice medicine independently. A health care professional may be any person that is permitted, authorized, registered or certified to collect a biological sample from a subject either independently or under the supervision of a medical practitioner. For example, the health care professional may be a registered or enrolled nurse, or a medical practitioner's assistant or a clinical assistant. It would be understood that the biological sample may, for example, be collected during routine outpatient procedures that would ordinarily be carried out on a patient who is under the clinical care of a medical practitioner.

Where the biological sample is urine, the sample may be collected under the supervision, directly or indirectly, of the medical practitioner with overall clinical care of the patient, but physically collected by the patient themselves.

In a particular embodiment, the method of the present invention is performed by a medical practitioner or by a person or persons under the supervision of a medical practitioner, or by a combination thereof. A person under the supervision of a medical practitioner may be, for example, a health care professional, a pharmacist, a clinical, medical or pathology laboratory technician, or a scientist. It would be understood that the method of the present invention may be performed in any laboratory by a medical practitioner or by a person or persons under the supervision of a medical practitioner, or by a combination thereof. For example, the collection, preparation and or testing of the biological sample(s) may be performed in a different location to the location at which a therapeutic dose is administered to a patient. Similarly, collection of the adipose tissue, preparation of the adipose tissue-derived cell suspension and administration may be performed by different individuals and may be performed at different locations. Where that occurs, typically all steps are performed by or under the supervision of a registered medical practitioner having prime responsibility for the clinical care of said patient throughout said method.

Testing the biological sample

Methods for testing a biological sample for the presence of a biomarker, such as those mentioned herein, are known in the art. For example, a sample potentially comprising a biomarker of interest can be contacted with an antibody that specifically binds the biomarker polypeptide or fragment thereof. Optionally, the antibody can be fixed to a solid support to facilitate washing and subsequent isolation of the complex, prior to contacting the antibody with a sample. Examples of solid supports include, for example, microtitre plates, beads, ticks, or microbeads. Antibodies can also be attached to a Protein-Chip array or a probe substrate as known in the art.

Useful assays for detecting the presence of or measuring the amount of, an antibody-marker complex include, include, for example, enzyme-linked immunosorbent assay (ELISA), a lateral flow assay, a radioimmune assay (RIA), or a Western blot assay. Such methods are described in, for example, Clinical Immunology (Stites & Terr, eds., 7th ed. 1991); Methods in Cell Biology: Antibodies in Cell Biology, volume 37 (Asai, ed. 1993); and Harlow & Lane, supra.

The method may include detection of whole proteins, peptides or fragments thereof. Hence it will be understood that reference herein to determining the level of a biomarker, or similar wording thereto, is intended to encompass the situation where the biomarker in its entirety is detected as well as the situation where a fragment of the biomarker, such as a peptide fragment, is detected except where the context indicates otherwise. Where the biomarker itself is breakdown product of a complete protein, however, the reverse does not apply. Hence, by way of clarification, where the biomarker is CTX-II, which is a fragment of collagen, reference to determining the level of CTX-II is not intended to encompass determining the level of collagen. The method may also further comprise the inclusion of controls, such as for the correct or consistent performance of the method.

In the case of CTX-II for example, CTX-II is corrected using creatinine concentration and may be expressed as ng/mmol of creatinine levels observed in a patient. In the subjects contributing the Example herein the observed levels were between about 100 to about 1500 ng/mmol.

The quantification of COMP and MIF was performed using ELISA assays from R&D Systems. (CTX-II) using the Urine Pre-clinical Cartilaps® (CTX-II) ELISA (Immunodiagnosticsystems, UK) assay according to the manufacturers instructions. CTX-II levels were corrected for creatinine (Cr) using the Creatinine Parameter Assay (R&D systems, USA). MIF ELISA conducted as described in the instructions (http://www.rndsystems.com/Products/SMF00B) and in Alexander et al. Exp Neurol. 2012 August; 236(2): 351-362. COMP and CTX-II ELISA assays conducted as described in the instructions (http://www.rndsystems.com/Products/DCMP0) and in V.B. Kraus et al. / Osteoarthritis and Cartilage 19 (2011). Particularly Table II, which contains references for a number of papers that describe ELISA assays for COMP and CTX-II, the contents of each of which are incoporated herein by reference.

*Blood collection.* At desired time-points, 8-36 mL of blood may be collected using 18 gauge needles from patients into BD vacutainers containing EDTA (Becton Dickinson, USA). Blood components may be separated using ficoll density gradient separation. Complete protease inhibitor cocktail (Roche, Switzerland) is added to the plasma at a 1:50 dilution and samples are transported on ice to the laboratory. The plasma is centrifuged at 16 000 x g for 5mins to remove any cellular material, aliquoted and stored at -80°C.

*Bio-Plex Analysis.* The Bio-Plex suspension array system (Bio-Rad, USA) uses unique fluorescently coloured beads to allow simultaneous quantitation of up to 100 analytes in a single well. Target analytes are quantified by detection of the unique bead conjugated to the primary antibody and the corresponding reporter complex using the Bio-Plex dual-laser flow-based microplate reader system. Samples are filtered through 0.2 μm Nanosep MF Centrifugal Devices with Bio-Inert® Membrane (Pall Scientific, USA) for 5 minutes at 9000 x g. 50 uL of each filtered sample is analysed using the desired Bio-Plex assay (Bio-Rad, USA) according to the manufacturer's instructions. The Bio-Plex Pro II magnetic wash station may be used for the washing steps and the data may be acquired using the Bio-Plex 200 system with version 5.0 software (Bio-Rad, USA).

The invention will now be described in more detail, by way of illustration only, with respect to the following examples. The examples are intended to serve to illustrate this invention and should not be construed as limiting the generality of the disclosure of the description throughout this specification.

## Kits and compositions

The invention also provides for kits comprising one or more components useful in the performance of the methods of the invention. For example, a pharmaceutical composition, such as comprising MSCs, or an adipose tissue-derived cell suspension, may be provided as part of a kit, for example including additional components useful in the intended treatment, such as for example written instructions, or it may be provided as a single item, such as a single vial or aliquot of the composition.

A kit may comprise one or more agents for the collection or preparation of a biological sample, or for the detection of a particular biomarker. For example, a kit may comprise one or more agent(s) capable of binding and or detecting, preferably capable of specifically binding and or detecting, MIF and optionally one or more biomarkers selected from the group consisting of CTX-II and COMP. A kit may comprise one or more agents for use in quantifying the level of MIF and optionally a biomarker, such as any one or more of CTX-II and COMP, in a biological sample.

A kit may be a compartmentalised kit, which terminology includes any kit in which reagents are contained in separate containers, and may include small glass containers, plastic containers or strips of plastic or paper. Such containers may allow the efficient transfer of reagents from one compartment to another compartment whilst avoiding cross-contamination of the samples and reagents, and the addition of agents or solutions of each container from one compartment to another in a quantitative fashion. Such kits may also include a container which will accept the test sample, a container which contains the antibody(s) used in the assay, containers which contain wash reagents (such as phosphate buffered saline, Tris-buffers, and like), and containers which contain the detection reagent. Different components of a kit may be presented, stored or transported at different temperatures.

## Examples

### Example 1: Osteoarthritis Stem Cell Advanced Research Study

The Osteoarthritis Stem Cell Advanced Research Study (OSCARS) was a randomised, double-blind, placebo-controlled trial to evaluate the safety and efficacy of autologous adipose-derived cell therapy for the treatment OA. A total of 40 patients were randomised (20:20) to receive a single intra-articular injection of autologous adipose-derived cells or placebo into their test knee. Participants completed self-reported pain questionnaires (Intermittent and Continuous Osteoarthritis Pain [ICOAP] index). The extent of cartilage degradation was assessed at baseline and 6 months post-treatment using MRI T2 mapping and cartilage degradation was measured using a cartilage specific collagen fragment (CTX-II) in urine and a panel of 48 cytokines in serum.

All patients experienced a large and significant reduction in their total pain scores that was maintained throughout the trial. MRI T2 mapping demonstrated that autologous adipose-derived cell therapy may have a disease modifying effect by slowing cartilage degradation even in subjects with severe cartilage damage. Consistent with this finding, CTX-II levels decreased in the treatment group but increased significantly (p=0.04) in the placebo group between baseline and 6 months. A significant reduction in the serum level of macrophage migration inhibitory factor (MIF), a key cytokine involved in cartilage degradation pathway, was observed in the treatment group.

OSCARs was a world-first trial designed to evaluate the effect of an autologous adipose-derived cell therapy for reducing knee pain in OA sufferers. The results in this interim report demonstrate that short to medium term symptom modification was similar in both the placebo and treatment groups. The objective markers of cartilage degradation were reduced in the treatment group and this was supported by MRI T2 mapping, which indicates that autologous adipose-derived cell therapy may slow the progression of OA and produce improved outcomes in the longer term.

A more detailed explanation of the manner in which the study was conducted, and the results and ramifications of the study is presented below.

Trial design

OSCARs was an ethics approved phase II, double-blind, placebo-controlled trial performed at Royal North Shore Hospital in Sydney, Australia. Patients age >40 years were eligible to enter the trial if they had diagnosed knee osteoarthritis, graded as Osteoarthritis Research Society International (OARSI) grade 1 or 2 radiographic joint space narrowing in either medial or lateral compartments or osteophyte grade 2 or 3 in medial or lateral compartment without joint space narrowing, and symptomatic knee osteoarthritis pain of at least 4 on a numerical rating scale (NRS). A total of 40 patients were randomly assigned (1:1) to receive a single intra-articular injection of autologous adipose-derived cells or placebo into their test knee.

The study objective was to determine whether in patients with diagnosed knee osteoarthritis, an injection of autologous non-expanded adipose-derived stem cells improved pain and altered disease progression. The primary objective was to determine the efficacy of using autologous stem cells to reduce pain symptoms in knee osteoarthritis. The secondary objectives were: (a) To determine the medium-term safety of using autologous stem cells in the treatment of knee osteoarthritis; (b) To evaluate the impact of an injection of autologous stem cells on biomarkers of disease progression; (c) To determine the impact of using autologous stem cells on quality of life.

Methodology

All participants underwent a routine liposuction procedure to harvest approximately 200 g of adipose tissue. The tissue was processed as described previously (Blaber SP, Webster RA, Hill CJ, et al. Analysis of in vitro secretion profiles from adipose-derived cell populations. J Transl Med 2012;10:172-88; the contents of which are incorporated herein by reference). Briefly, the adipose tissue was digested with collagenase and centrifuged to obtain the pelleted cells (SVF) and the adipocytes. The SVF and adipocyte cell suspension was washed twice with saline. The resultant mixed cell population was resuspended in saline to a final volume of 5mL. The treatment group received an intra-articular injection of this cell suspension into their test knee by an independent radiologist whereas the placebo group received an intra-articular injection of saline. The participants and the investigators remained blinded to the treatment allocation throughout the trial. Patients were monitored for adverse events (AEs) and concomitant medications throughout the study.

Participants completed self-reported pain questionnaires (Intermittent and Continuous Osteoarthritis Pain [ICOAP] index) at 1 month, 3 months, 6 months, and 12 months. Cartilage quality was assessed at baseline and 6-months post-treatment using MRI T2 mapping. Secondary outcome measures included assessment of urine and serum biomarkers at baseline, 1 month and 6-months post-treatment. CTX-II was measured in urine at Royal North Shore Hospital using an ELISA kit. Urinary creatinine levels were also measured by ELISA and all CTX-II data shown are creatinine corrected. Serum was analysed for a panel of 48 cytokines using Bio-Rad Bio-Plex kits at the Australian Proteome Analysis Facility (APAF) at Macquarie University. Data was analysed using intention-to-treat principles.

Results and Discussion

This study demonstrated that the autologous adipose-derived cells treatment was safe and clinically feasible. The treatment was well tolerated by patients and there were no major medium-term safety concerns and no joint infections.

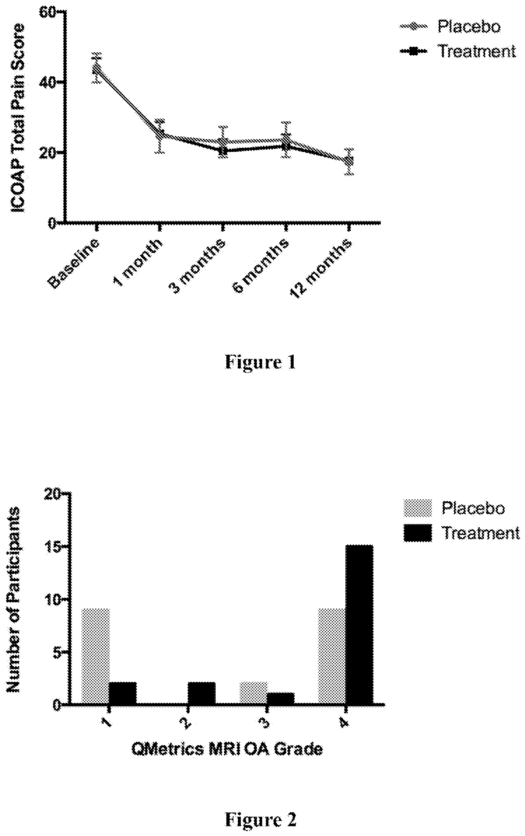

Both the placebo and autologous adipose-derived cells treatment groups experienced a large and statistically significant decrease in their total pain scores from baseline, as measured by ICOAP, which was maintained to the final follow-up time-point of 12 months (**Figure 1**). A significant effect in the placebo group was not unexpected, as it has been established that placebo has a treatment effect in OA sufferers in terms of self-reported outcomes, in particular pain.

*Assessment of cartilage degradation by MRI.* Patients were screened for inclusion into this trial by radiographic imaging of both knees. All patients were graded as Osteoarthritis Research Society International (OARSI) grade 1 or 2 joint space narrowing in either medial or lateral compartments or osteophyte grade 2 or 3 in medial or lateral compartment without joint space narrowing, and randomised into the placebo or treatment groups. Radiographic imaging of joints provides an indirect measure of articular cartilage quality by joint space narrowing but does not provide soft tissue information. MRI is a powerful and sensitive imaging technique which enables an assessment of cartilage morphology and physiology. MRI T2 mapping enables a quantitative assessment of the molecular content and structure of the cartilage. In particular, T2 mapping assesses the collagen bundle orientation and integrity of the proteoglycan-collagen matrix, as well as the extent of cartilage damage through water content.

MRI T2 mapping was performed on the test knee of all participants at baseline and 6 months post-treatment. The analysis was performed by Qmetrics Technologies, an independent contract research organisation that specialises in MRI imaging. The T2 mapping revealed that loss of cartilage was slower than expected at the 6-month post-treatment time-point. It was found that both groups exhibited a greater proportion of subjects' remaining stable than those progressing. Although all participants had OARSI joint space narrowing grade 1 or 2, a detailed assessment of cartilage damage at baseline was not part of the inclusion criteria.

The MRI analysis showed that at baseline there were significantly more participants with advanced cartilage damage in the autologous adipose-derived cells treatment group, that is there were significantly (p 0.03) more grade 4 OA patients in the treatment group than the placebo group. **Figure 2** shows the QMetrics assessment of cartilage damage at baseline by MRI, which is ranked by OA grade. This would tend to predispose the autologous adipose-derived cells treatment group toward an accelerated progression of OA, hence would make it less likely that significant differences would be observed between the groups, however this was not observed. This result suggests that autologous adipose-derived cells therapy may have a disease modifying effect by slowing cartilage degradation.

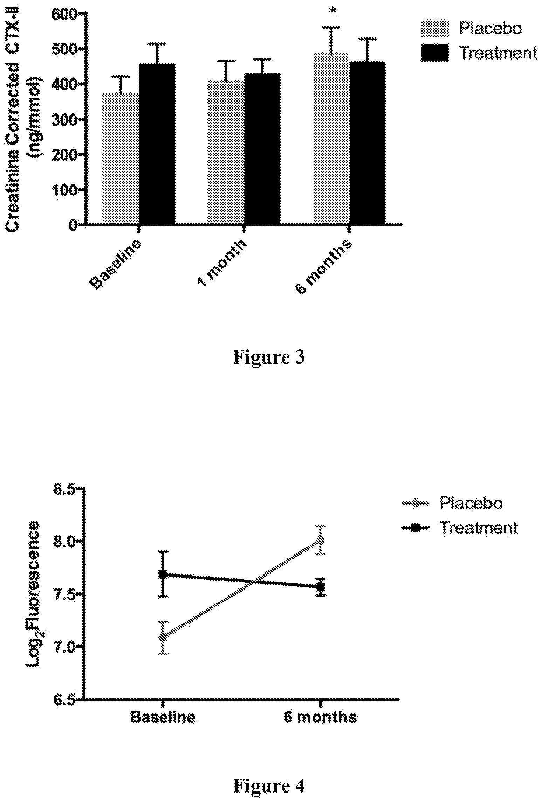

*Analysis of disease progression by assessment of OA biomarkers.* To investigate the effects of autologous adipose-derived cells therapy at the molecular level, OA biomarkers were measured in the participant's urine and serum at baseline, 1 month and 6 months post-treatment. CTX-II is a C-terminal telopeptide of type II collagen and is a non-invasive marker of cartilage damage measured in urine. In this trial, a 31% increase in urinary CTX-II was observed between baseline and 6 months in the placebo group (p=0.04; **Figure 3**). The average CTX-II level decreased in the autologous adipose-derived cells treatment group over the course of the trial. This result was unexpected given the MRI analysis indicated this group contained significantly more subjects with advanced cartilage damage at baseline. This result correlates with the Qmetrics T2 mapping MRI results indicating that autologous adipose-derived cells slows cartilage degradation even in subjects with advanced cartilage damage.

Whilst osteoarthritis was historically referred to as a non-inflammatory disease, it is now increasingly evident that low-grade inflammation plays a major role in OA disease progression. OA involves an imbalance between degradation and repair mechanisms and affects the entire joint structure. To investigate the effect of autologous adipose-derived cells therapy on inflammation, a panel of 48 cytokines, chemokines and growth factors were measured and analysed in the serum of participants at APAF (Macquarie University).

The analysis indicated that autologous adipose-derived cells treatment reduced inflammation at both 1 and 6 months compared to the placebo group. The key cytokine was macrophage migration inhibitory factor (MIF), which was significantly reduced in the autologous adipose-derived cells treatment group at 6months (p=0.00; **Figure 4**). MIF is produced by a number of cells including T cells and synovial fibroblasts. MIF is detected in higher levels in the serum and synovial fluid of patients with knee OA than healthy controls. MIF induces the up-regulation of the matrix metalloproteinases MMP-1 and MMP-3 in synovial fibroblasts in a concentration and time dependent manner. MMPs have a primary role in the destruction of cartilage. Therefore, the reduction of circulating MIF levels in the autologous adipose-derived cells treatment group is likely to have reduced cartilage degradation via decreased enzyme levels.

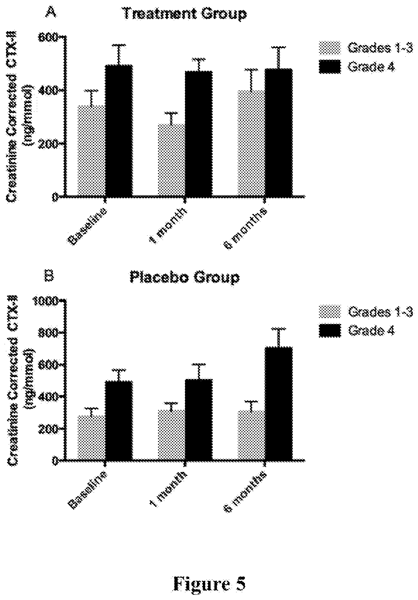

*Stratification of CTX-II results by MRI derived OA grades.* Osteoarthritis commonly develops bilaterally in the knees of people, but can also affect other joints in the body. The OSCARs trial was designed to treat unilateral knee OA with autologous adipose-derived cells therapy or a placebo injection of saline. However, the baseline radiographic screening illustrated that the majority of participants also had evidence of OA in their untreated knee. In some cases their untreated knee had more advanced joint space narrowing and osteophyte scores than their test knee. As the OA biomarkers investigated in this study were measured in the serum and urine of the trial participants, the extent of damage in the untreated knee and other arthritic joints will have contributed to these results. In order to determine whether the levels of CTX-II are increased in subjects with advanced cartilage degradation, the results were stratified by OA grade assessed by MRI. **Figures 5A** and **5B** show the CTX-II values for placebo and autologous adipose-derived cells treatment groups stratified by the MRI derived OA grades.

In the autologous adipose-derived cells treatment group, the 15 grade 4 OA patients had an average CTX-II level of 491 ng/mmol at baseline, which reduced to 477 ng/mmol at 6 months. The 9 placebo grade 4 OA patients CTX-II levels increased from an average of 490 ng/mmol at baseline to 702 ng/mmol at 6 months, an increase of 43% (p=0.037 using a one-tailed t-test; p=0.074 using a two-tailed t-test). Comparing the grade 4 OA patients from test and placebo at six months post treatment, the 15 test patients have an average of 477 ng/mmol and the 9 placebo patients, 702 ng/mmol (p-value=0.066 using a one-tailed t-test). This result indicates that cartilage degradation in the placebo group with advanced cartilage damage increased over the course of the trial. In contrast, cartilage degradation was slowed in cohort of participants in the autologous adipose-derived cells treatment group with an equivalent degree of cartilage damage. Taken together, these results support that autologous adipose-derived cells treatment may have a disease modifying effect for the treatment of OA.

OSCARs was a world-first trial designed to evaluate the effect of an autologous adipose-derived cell therapy for reducing knee pain in OA sufferers. The treatment process was well tolerated and there were no major medium-term safety concerns. The results in demonstrate that short to medium term symptom modification was similar in both the placebo and treatment groups. The objective markers of cartilage degradation were reduced in the treatment group and this was supported by MRI T2 mapping, which indicates that autologous adipose-derived cells treatment may slow the progression of OA and produce improved outcomes in the longer term.

## Example 2: Treatment of a neurodegenerative disease with MSCs

Two 4 year old children with a neurodegenerative disease (monocarboxylate transporter 8 (MCT8) deficiency) were treated once in 2010 and once in 2011 with culture expanded allogeneic umbilical cord blood derived MSCs. Cells were administered intravenously.

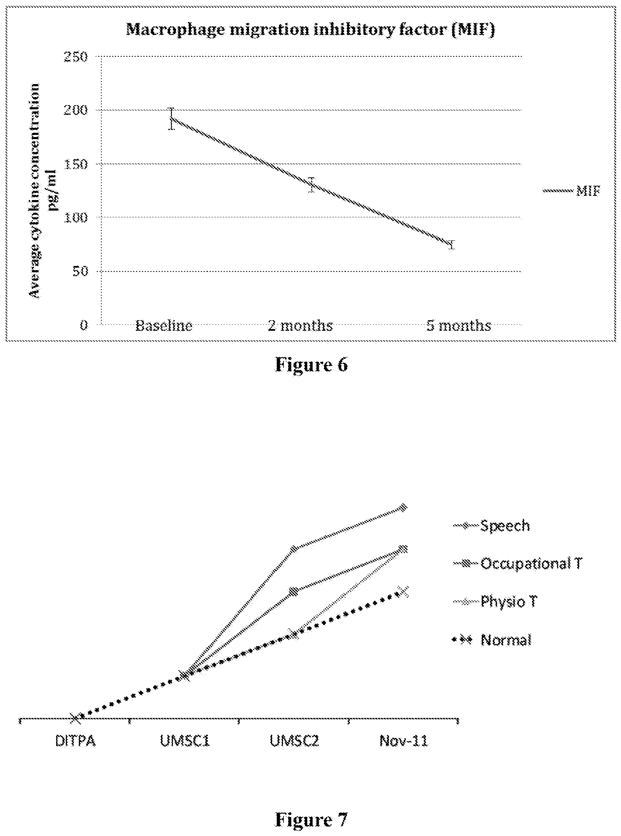

**Figure 6** shows baseline MIF levels before the second MSC treatment in 2011 and MIF at 2 months and 5 months post-treatment. The decrease in MIF correlates with improvements in neurological and physical tests undertaken by the boys.

The boys are constantly assessed by therapists and their scores recorded for various neurological and physical tasks (**Figure 7**). They showed unexpected gains after each MSC treatment. Speech and occupational therapy showed a dramatic improvement after the first MSC treatment. Their ability to undertake physical tasks showed marked improvement after the second MSC treatment.

**Example 3: Treatment of musculoskeletal conditions with autologous adipose-derived cells**

The dataset referred to in this example include osteoarthritis patients from the HiQCell Joint Registry. The registry protocol has been approved by Bellberry Human Research Ethics Committee and is registered with the Australian and New Zealand Clinical Trials Registry. The HiQCell Treating Medical Practitioners (TMPs) are co-investigators on the study and include orthopaedic surgeons and sports physicians trained in the diagnosis and treatment of musculoskeletal conditions. This is an observational registry with unlimited patient recruitment. Unlike a randomised, controlled clinical trial, there is no inclusion or exclusion criteria so all patients undergoing HiQCell treatment with a Regeneus-accredited TMP are eligible for inclusion, resulting in a heterogeneous population. Serum and urine samples were also collected from HiQCell patients outside of the HiQCell Joint Registry.

Methodology

All participants underwent a routine liposuction procedure to harvest approximately 100-200 g of adipose tissue. The tissue was processed as described previously (Blaber SP, Webster RA, Hill CJ, et al. Analysis of in vitro secretion profiles from adipose-derived cell populations. J Transl Med 2012;10:172-88; the contents of which are incorporated herein by reference). Briefly, the adipose tissue was digested with collagenase and centrifuged to obtain the pelleted cells (SVF) and the adipocytes. The SVF and adipocyte cell suspension was washed twice with saline. The resultant mixed cell population was resuspended in saline to a final volume of between 5mL-10mL depending on the number of joints to be injected. A proportion of the mixed cell population was injected into each of the affected joints.

In a subset of fifteen patients, outcome measures included assessment of urine and serum biomarkers at baseline, and monthly timepoints post-treatment. CTX-II was measured in urine at the Australian Proteome Analysis Facility (APAF) at Macquarie University using an ELISA kit. Urinary creatinine levels were also measured by ELISA and all CTX-II data shown are creatinine corrected. Serum was analysed for cartilage oligomeric matrix protein (COMP) and MIF. COMP is a recognised biomarker of osteoarthritis and associated cartilage degradation, with serum levels significantly elevated in OA patients. (J Orthop Res. 2013 Jul;31(7):999-1006. doi: 10.1002/jor.22324. Epub 2013 Feb 19, Serum cartilage oligomeric matrix protein (COMP) in knee osteoarthritis: a novel diagnostic and prognostic biomarker).

Results

As at July-2014, a total of 386 patients were included in the Joint Registry, representing 78% of the total number of 494 patients treated with HiQCell. The average pain score for all treated joints reduced at every post-treatment time-point: by 24% at 2 weeks; 46% at 6 months; 49% at 1 year and by 51% at 2 years post-treatment.

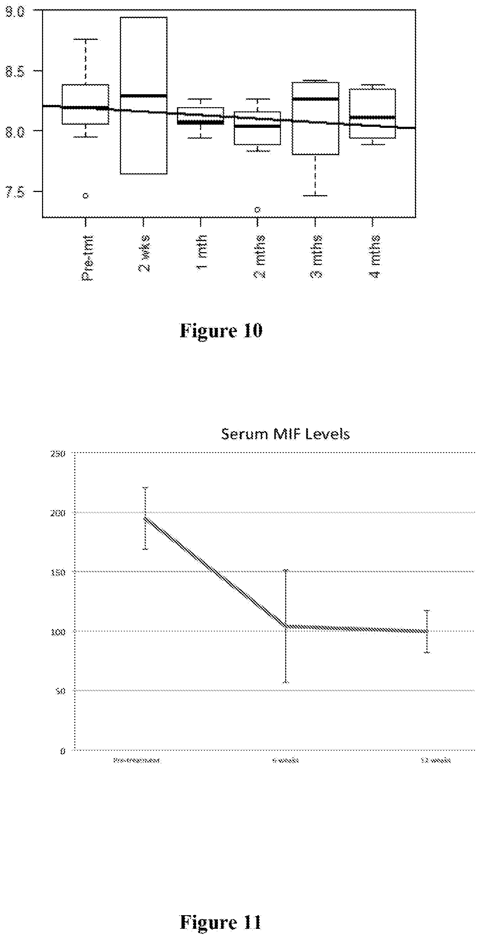

Figure 8 shows a boxplot of serum MIF levels from pre-treatment to 4 months post-treatment. The trend analysis shows a significant (p 0.001) decrease in MIF levels over the 4 months post-treatment. Figures 9 and 10 show boxplots and trendlines for CTXII and COMP from pre-treatment to 4 months post-treatment. The trend is similar to that observed in the OSCARS study (Example 1) and indicates that cartilage degradation was slowed in the 15 participants analysed from the HiQCell joint registry. Taken together, these results support that autologous adipose-derived cells treatment may have a disease modifying effect for the treatment of OA

**Example 4: Treatment of ulcerative colitis with MSCs**

Two adults having ulcerative colitis, a form of inflammatory bowel disease (IBD), were treated with umbilical cord MSCs. Ulcerative colitis is characterised by inflammation and multiple ulcers of the large intestine. The individuals were each treated in 2013 with a single dose of culture expanded allogeneic umbilical cord blood derived MSCs, administered intravenously. **Figure 11** shows a reduction in measurable MIF when assessed at 6 weeks and 12 weeks post-treatment. Cell therapy was effective at reducing inflammation (as measured by detectable MIF in serum) and reducing the symptoms of the disease.

As demonstrated in the Examples herein, the correlation of the levels of the objective biomarkers determined in biological samples from the patients with the condition provides the basis for a method by which to assess the status of the condition in the patient, for example to assist the treating physician to determine an appropriate time to administer a therapeutic dose to the patient.

**Claims**

1. A method for monitoring disease progression in a patient having mesenchymal stem cell therapy for an inflammatory condition, the method comprising determining the level of a biomarker MIF in at least a first and a second biological sample from said patient, wherein a change in the level of said biomarker in said second compared to said first biological sample is indicative of disease progression, wherein (i) an increase in the detectable level of said biomarker in a second compared to a first biological sample is indicative of pathological progression, or deterioration, of the inflammatory condition; or (ii) a decrease in the detectable level of said biomarker in a second compared to a first biological sample is indicative of stabilisation of or improvement of the inflammatory condition, and wherein said therapy comprises

(i) a course of treatment comprising multiple doses over time of a cell suspension comprising mesenchymal stem cells (MSCs); or (ii) a course of treatment comprising multiple doses over time of an autologous adipose tissue-derived cell suspension comprising adipose tissue-derived non-adipocyte cells, wherein a first dose comprises a portion of a freshly prepared cell suspension and a subsequent dose or doses comprise a portion of said cell suspension that has been stored frozen; or (iii) a course of treatment comprising multiple doses over time of an allogeneic adipose tissue-derived cell suspension comprising adipose tissue-derived non-adipocyte cells, wherein all doses comprise a cell suspension that has been stored frozen.

2. The method of claim 1, wherein (i) the first biological sample is a baseline sample from said patient prior to commencement of said therapy and the second biological sample is a sample from said patient after a first dose of mesenchymal stem cells, wherein if the level of the biomarker MIF is approximately the same in the second sample compared to the first sample, determining that a further dose of mesenchymal stem cells is to be administered, or (ii) both the first and the second biological samples are from said patient after a first dose of mesenchymal stem cells, wherein if an increase in the level of the biomarker MIF is determined in the second sample compared to the first sample, determining that a further dose of the mesenchymal stem cells is to be administered.

3. The method according to claim 1 or 2, wherein the MSCs are selected from autologous cells, allogeneic cells, cord blood cells, and expanded cord blood cells, or a mixture thereof.

4. The method according to any one of claims 1 to 3, wherein (i) the inflammatory condition is a condition **characterised by** or associated with cartilage damage or degeneration; or (ii) the inflammatory condition is selected from the group consisting of osteoarthritis, rheumatoid arthritis and inflammatory bowel disease, such as ulcerative colitis.

5. The method according to any one of claims 1 to 4, wherein the inflammatory condition is selected from OA or a condition **characterised by** or associated with cartilage damage or degeneration, and the method further comprises determining the level of at least a second biomarker selected from CTX-II and COMP in said biological sample or samples.

6. A method for monitoring disease progression in a patient having mesenchymal stem cell therapy for a condition **characterised by** inflammation and tissue degradation, such as cartilage damage or degeneration, the method comprising determining the level of a biomarker MIF and optionally at least one biomarker selected from COMP and CTX-II in at least a first and a second biological sample from said patient, wherein a change in the level of said biomarker in said second compared to said first biological sample is indicative of disease progression, wherein said therapy comprises (i) a course of treatment comprising multiple doses over time of a cell suspension comprising mesenchymal stem cells (MSCs); or (ii) a course of treatment comprising multiple doses over time of an autologous adipose tissue-derived cell suspension comprising adipose tissue-derived non-adipocyte cells, wherein a first dose comprises a portion of a freshly prepared cell suspension and a subsequent dose or doses comprise a portion of said cell suspension that has been stored frozen; or (iii) a course of treatment comprising multiple doses over time of an allogeneic adipose tissue-derived cell suspension comprising adipose tissue-derived non-adipocyte cells, wherein all doses comprise a cell suspension that has been stored frozen and wherein (i) an increase in the detectable level of said biomarker in a second compared to a first biological sample is indicative of pathological progression, or deterioration, of the condition; or (ii) a decrease in the detectable level of said biomarker in a second compared to a first biological sample is indicative of stabilisation of or improvement of the condition.

7. The method according to claim 6, wherein (i) the condition **characterised by** cartilage damage or degeneration is a chronic condition; or (ii) the condition **characterised by** cartilage damage or degeneration is arthritis, such as osteoarthritis (OA) or rheumatoid arthritis (RA); or (iii) the condition **characterised by** cartilage damage or degeneration is an acute condition, such as injury or trauma to a cartilage.

8. The method according to any one of claims 1 to 7, wherein the method comprises (i) determining the level of two or more of COMP, CTX-II and MIF in at least a first and a second biological sample from said patient; or (ii) determining the level of COMP, CTX-II and MIF in at least a first and a second biological sample from said patient.

9. The method according to any one of claims 1 to 8, wherein the first biological sample is a baseline sample from said patient prior to commencement of said therapy.

10. The method according to any one of claims 1 to 9, wherein (i) the second biological sample is a sample from said patient after commencement of a treatment for the condition; or(ii) the second biological sample is a sample from said patient one week to twelve months after commencement of a treatment for the condition; or (iii) the second biological sample is a sample from said patient at least one month after administration of MSCs; or (iv) the second biological sample is a sample from said patient at least three months after administration of MSCs; or (v) both the first and the second biological samples are samples from the patient after commencement of the therapy.

11. The method according to any one of claims 1 to 10, wherein the first biological sample is a sample from said patient prior to commencement of a treatment for said condition and the second biological sample is a sample from said patient after commencement of the treatment, wherein if the determined level of said at least one biomarker in said first and said second samples is approximately the same, determining that a further dose of mesenchymal stem cells is to be administered.

12. The method according to any one of claims 1 to 11, wherein the method further comprises determining the level of said biomarker in additional biological samples from said patient, such as a third, fourth, fifth, etc biological sample, wherein each of said biological samples is from said patient at different times before and or after commencement of therapy for the condition.

13. The method according to any one of claims 1 to 12, wherein the biological sample is a circulating blood sample or fraction thereof.

**Patentansprüche**

1. Verfahren zum Überwachen des Krankheitsverlaufs bei einem Patienten mit mesenchymaler Stammzelltherapie für einen entzündlichen Zustand, wobei das Verfahren das Bestimmen des Wertes eines MIF Biomarker in mindestens einer ersten und einer zweiten biologischen Probe des Patienten umfasst, wobei eine Änderung des Wertes des Biomarkers in der zweiten verglichen mit der ersten biologischen Probe auf das Fortschreiten der Krankheit hinweist, wobei (i) ein Anstieg des nachweisbaren Wertes des Biomarkers in einer zweiten verglichen zu einer ersten biologischen Probe auf eine pathologische Progression oder Verschlechterung des Entzündungszustandes hinweist; oder (ii) ein Rückgang des nachweisbaren Wertes des Biomarkers in einer zweiten verglichen zu einer ersten biologischen Probe auf eine Stabilisierung oder Verbesserung des Entzündungszustandes hinweist, und wobei die Therapie umfasst

(i) einen Behandlungsverlauf, der im Laufe der Zeit mehrere Dosen einer Zellsuspension, umfassend mesenchymale Stammzellen (MSCs), umfasst; oder (ii) einen Behandlungsverlauf, der im Laufe der Zeit mehrere Dosen einer autologen, aus Fettgewebe abgeleiteten Zellsuspension umfasst, die aus Fettgewebe abgeleitete, nichtadipozytäre Zellen umfasst, wobei eine erste Dosis einen Teil einer frisch hergestellten Zellsuspension umfasst und eine nachfolgende Dosis oder mehrere Dosen einen Teil der Zellsuspension umfassen, der gefroren gelagert wurde; oder (iii) einen Behandlungsverlauf, der im Laufe der Zeit mehrere Dosen einer allogenen, aus Fettgewebe gewonnenen Zellsuspension umfasst, die aus Fettgewebe gewonnene, nichtadipozytäre Zellen umfasst, wobei alle Dosen eine Zellsuspension umfassen, die eingefroren gelagert wurde.

2. Das Verfahren gemäß Anspruch 1, wobei (i) die erste biologische Probe eine Basisprobe des Patienten vor Beginn der Therapie ist und die zweite biologische Probe eine Probe des Patienten nach einer ersten Dosis mesenchymaler Stammzellen ist, wobei, wenn der Wert des MIF Biomarkers in der zweiten Probe im Vergleich zur ersten Probe ungefähr gleich ist, bestimmt, dass eine weitere Dosis mesenchymaler Stammzellen verabreicht werden soll, oder (ii) sowohl die erste als auch die zweite biologische Probe von dem Patienten nach einer ersten Dosis mesenchymaler Stammzellen stammen, wobei, wenn in der zweiten Probe eine Erhöhung des Wertes des MIF Biomarkers im Vergleich zur ersten Probe bestimmt wird, bestimmt, dass eine weitere Dosis der mesenchymalen Stammzellen zu verabreichen ist.

3. Das Verfahren gemäß Anspruch 1 oder 2, wobei die MSCs ausgewählt sind aus autologen Zellen, allogenen Zellen, Nabelschnurblutzellen und expandierten Nabelschnurblutzellen oder einer Mischung davon.

4. Das Verfahren gemäß einem der Ansprüche 1 bis 3, wobei (i) der Entzündungszustand ein Zustand ist, der durch einen Knorpelschaden oder eine Degeneration gekennzeichnet ist oder damit verbunden ist; oder (ii) der Entzündungszustand ausgewählt ist aus der Gruppe bestehend aus Osteoarthrose, rheumatoider Arthritis und entzündlicher Darmerkrankung, wie z.B. Colitis ulcerosa.

5. Das Verfahren gemäß einem der Ansprüche 1 bis 4, wobei der Entzündungszustand ausgewählt ist aus OA oder einem Zustand, der durch Knorpelschäden oder Degeneration gekennzeichnet ist oder damit verbunden ist, und das Verfahren ferner das Bestimmen des Wertes von mindestens einem zweiten Biomarker in der oder den biologischen Proben umfasst, ausgewählt aus CTX-II und COMP.

6. Verfahren zum Überwachen des Krankheitsverlaufs bei einem Patienten mit mesenchymaler Stammzelltherapie für einen durch Entzündung und Gewebeabbau gekennzeichneten Zustand, wie Knorpelschaden oder Degeneration, wobei das Verfahren das Bestimmen des Wertes eines MIF Biomarker und gegebenenfalls mindestens eines Biomarkers, ausgewählt aus COMP und CTX-II, in mindestens einer ersten und einer zweiten biologischen Probe des Patienten umfasst, wobei eine Änderung des Wertes des Biomarkers in der zweiten verglichen mit der ersten biologischen Probe auf das Fortschreiten der Krankheit hinweist,
wobei die Therapie umfasst (i) einen Behandlungsverlauf, der im Laufe der Zeit mehrere Dosen einer Zellsuspension, umfassend mesenchymale Stammzellen (MSCs), umfasst; oder (ii) einen Behandlungsverlauf, der im Laufe der Zeit mehrere Dosen einer autologen, aus Fettgewebe abgeleiteten Zellsuspension umfasst, die aus Fettgewebe abgeleitete, nichtadipozytäre Zellen umfasst, wobei eine erste Dosis einen Teil einer frisch hergestellten Zellsuspension umfasst und eine nachfolgende Dosis oder mehrere Dosen einen Teil der Zellsuspension umfassen, der gefroren gelagert wurde; oder (iii) einen Behandlungsverlauf, der im Laufe der Zeit mehrere Dosen einer allogenen, aus Fettgewebe gewonnenen Zellsuspension umfasst, die aus Fettgewebe gewonnene, nichtadipozytäre Zellen umfasst, wobei alle Dosen eine Zellsuspension umfassen, die eingefroren gelagert wurde und wobei (i) ein Anstieg des nachweisbaren Wertes des Biomarkers in einer zweiten verglichen zu einer ersten biologischen Probe auf eine pathologische Progression oder Verschlechterung des Zustandes hinweist; oder (ii) ein Rückgang des nachweisbaren Wertes des Biomarkers in einer zweiten verglichen zu einer ersten biologischen Probe auf eine Stabilisierung oder Verbesserung des Zustandes hinweist.

7. Das Verfahren gemäß Anspruch 6, wobei (i) der durch Knorpelschaden oder Degeneration gekennzeichnete Zustand ein chronischer Zustand ist; oder (ii) der durch Knorpelschaden oder Degeneration gekennzeichnete Zustand eine Arthritis, wie Osteoarthrose (OA) oder rheumatoide Arthritis (RA), ist; oder (iii) der durch Knorpelschaden oder Degeneration gekennzeichnete Zustand ein akuter Zustand, wie Verletzung oder Trauma eines Knorpels ist.

8. Das Verfahren gemäß einem der Ansprüche 1 bis 7, wobei das Verfahren umfasst: (i) Bestimmen des Wertes von zwei oder mehr von COMP, CTX-II und MIF in mindestens einer ersten und einer zweiten biologischen Probe des Patienten; oder (ii) Bestimmen des Wertes von COMP, CTX-II und MIF in mindestens einer ersten und einer zweiten biologischen Probe des Patienten.

9. Das Verfahren gemäß einem der Ansprüche 1 bis 8, wobei die erste biologische Probe eine Basisprobe des Patienten vor Beginn der Therapie ist.

10. Das Verfahren gemäß einem der Ansprüche 1 bis 9, wobei (i) die zweite biologische Probe eine Probe des Patienten nach Beginn einer Behandlung für den Zustand ist; oder (ii) die zweite biologische Probe eine Probe des Patienten eine Woche bis zwölf Monate nach Beginn einer Behandlung für den Zustand ist; oder (iii) die zweite biologische Probe eine Probe des Patienten mindestens einen Monat nach Verabreichung von MSCs ist; oder (iv) die zweite biologische Probe eine Probe des Patienten mindestens drei Monate nach Verabreichung von MSCs ist; oder (v) sowohl die erste als auch die zweite biologische Probe Proben von dem Patienten nach Beginn der Therapie sind.

11. Das Verfahren gemäß einem der Ansprüche 1 bis 10, wobei die erste biologische Probe eine Probe des Patienten vor Beginn einer Behandlung für den Zustand ist und die zweite biologische Probe eine Probe des Patienten nach Beginn der Behandlung ist, wobei, wenn der bestimmte Wert des mindestens einen Biomarkers in der ersten und der zweiten Probe ungefähr gleich ist, bestimmt wird, dass eine weitere Dosis mesenchymaler Stammzellen verabreicht werden soll.

12. Das Verfahren gemäß einem der Ansprüche 1 bis 11, wobei das Verfahren ferner das Bestimmen des Wertes des Biomarkers in zusätzlichen biologischen Proben von dem Patienten, wie beispielsweise einer dritten, vierten, fünften usw. biologischen Probe, umfasst, wobei jede der biologischen Proben von dem Patienten zu unterschiedlichen Zeiten vor und nach Beginn der Therapie für den Zustand stammt.

**13.** Das Verfahren gemäß einem der Ansprüche 1 bis 12, wobei die biologische Probe eine Probe von zirkulierendem Blut oder eine Fraktion davon ist.

## Revendications

**1.** Procédé de suivi d'une progression de maladie chez un patient en thérapie par cellules souches mésenchymateuses pour un état inflammatoire, le procédé comprenant la détermination du niveau d'un biomarqueur MIF dans au moins un premier et un deuxième échantillon biologique issus dudit patient, sachant qu'un changement dans le niveau dudit biomarqueur dans ledit deuxième échantillon biologique comparé audit premier échantillon biologique est indicateur d'une progression de maladie,
sachant que (i) une hausse dans le niveau détectable dudit biomarqueur dans un deuxième échantillon biologique comparé à un premier échantillon biologique est indicatrice d'une progression pathologique, ou d'une détérioration, de l'état inflammatoire ; ou (ii) une baisse dans le niveau détectable dudit biomarqueur dans un deuxième échantillon biologique comparé à un premier échantillon biologique est indicatrice d'une stabilisation ou d'une amélioration de l'état inflammatoire, et sachant que ladite thérapie comprend

(i) un cours de traitement comprenant de multiples doses dans le temps d'une suspension cellulaire comprenant des cellules souches mésenchymateuses (MSC) ; ou (ii) un cours de traitement comprenant de multiples doses dans le temps d'une suspension cellulaire dérivée de tissu adipeux autologue comprenant des cellules non adipocytes dérivées de tissu adipeux, sachant qu'une première dose comprend une partie de suspension cellulaire fraîchement préparée et une dose ou des doses subséquentes comprennent une partie de ladite suspension cellulaire qui a été stockée en congélation ; ou (iii) un cours de traitement comprenant de multiples doses dans le temps d'une suspension cellulaire dérivée de tissu adipeux allogénique comprenant des cellules non adipocytes dérivées de tissu adipeux, sachant que toutes les doses comprennent une suspension cellulaire qui a été stockée en congélation.

**2.** Le procédé de la revendication 1, sachant que (i) le premier échantillon biologique est un échantillon de référence issu dudit patient avant le commencement de ladite thérapie et le deuxième échantillon biologique est un échantillon issu dudit patient après une première dose de cellules souches mésenchymateuses, sachant que si le niveau du biomarqueur MIF est approximativement le même dans le deuxième échantillon comparé au premier échantillon, on détermine qu'une dose supplémentaire de cellules souches mésenchymateuses doit être administrée, ou (ii) à la fois le premier et le deuxième échantillon biologique sont issus dudit patient après une première dose de cellules souches mésenchymateuses, sachant que si une hausse dans le niveau du biomarqueur MIF est déterminée dans le deuxième échantillon comparé au premier échantillon, on détermine qu'une dose supplémentaire des cellules souches mésenchymateuses doit être administrée.

**3.** Le procédé selon la revendication 1 ou 2, sachant que les MSC sont sélectionnées parmi des cellules autologues, des cellules allogéniques, des cellules de sang de cordon ombilical, et des cellules de sang de cordon ombilical expansées, ou un mélange de celles-ci.

**4.** Le procédé selon l'une quelconque des revendications 1 à 3, sachant que (i) l'état inflammatoire est un état **caractérisé par** ou associé à un endommagement ou une dégénérescence de cartilage ; ou (ii) l'état inflammatoire est sélectionné dans le groupe constitué par l'ostéoarthrite, l'arthrite rhumatoïde et l'affection abdominale inflammatoire, comme la colite ulcéreuse.

**5.** Le procédé selon l'une quelconque des revendications 1 à 4, sachant que l'état inflammatoire est sélectionné parmi l'OA ou un état **caractérisé par** ou associé à un endommagement ou une dégénérescence de cartilage, et le procédé comprend en outre la détermination du niveau d'au moins un deuxième biomarqueur sélectionné parmi CTX-II et COMP dans ledit ou lesdits échantillons biologiques.

**6.** Procédé de suivi d'une progression de maladie chez un patient en thérapie par cellules souches mésenchymateuses pour un état **caractérisé par** de l'inflammation et une dégradation de tissu, comme un endommagement ou une dégénérescence de cartilage, le procédé comprenant la détermination du niveau d'un biomarqueur MIF et facultativement d'au moins un biomarqueur sélectionné parmi COMP et CTX-II dans au moins un premier et un deuxième échantillon biologique issus dudit patient, sachant qu'un changement dans le niveau dudit biomarqueur dans ledit deuxième échantillon biologique comparé audit premier échantillon biologique est indicateur d'une progression de maladie, sachant que ladite thérapie comprend (i) un cours de traitement comprenant de multiples doses dans le temps d'une suspension cellulaire comprenant des cellules souches mésenchymateuses (MSC) ; ou (ii) un cours de traitement comprenant de multiples doses dans le temps d'une suspension cellulaire dérivée de tissu adipeux autologue comprenant des cellules non adipocytes dérivées de tissu adipeux, sachant qu'une première dose comprend une partie de suspension cellulaire fraîchement préparée et une dose ou des doses subséquentes comprennent une partie de ladite suspension cellulaire qui a été stockée en congélation ; ou (iii) un cours de traitement comprenant de multiples doses dans le temps d'une suspension cellulaire dérivée de tissu adipeux allogénique comprenant des cellules non adipocytes dérivées de tissu adipeux, sachant que toutes les doses comprennent une suspension cellulaire qui a été stockée en congélation et sachant que (i) une hausse dans le niveau détectable dudit biomarqueur dans un deuxième échantillon biologique comparé à un premier échantillon biologique est indicatrice d'une progression pathologique, ou d'une détérioration, de l'état ; ou (ii) une baisse dans le niveau détectable dudit biomarqueur dans un deuxième échantillon biologique comparé à un premier échantillon biologique est indicatrice d'une stabilisation ou d'une amélioration de l'état.

7.  Le procédé selon la revendication 6, sachant que (i) l'état **caractérisé par** un endommagement ou une dégénérescence de cartilage est un état chronique ; ou (ii) l'état **caractérisé par** un endommagement ou une dégénérescence de cartilage est de l'arthrite, comme l'ostéoarthrite (OA) ou l'arthrite rhumatoïde (RA) ; ou (iii) l'état **caractérisé par** un endommagement ou une dégénérescence de cartilage est un état aigu, comme une lésion ou un traumatisme du cartilage.

8.  Le procédé selon l'une quelconque des revendications 1 à 7, sachant que le procédé comprend (i) la détermination du niveau de deux ou plus de deux de COMP, CTX-II et MIF dans au moins un premier et un deuxième échantillon biologique issus dudit patient ; ou (ii) la détermination du niveau de COMP, CTX-II et MIF dans au moins un premier et un deuxième échantillon biologique issus dudit patient.

9.  Le procédé selon l'une quelconque des revendications 1 à 8, sachant que le premier échantillon biologique est un échantillon de référence issu dudit patient avant le commencement de ladite thérapie.

10. Le procédé selon l'une quelconque des revendications 1 à 9, sachant que (i) le deuxième échantillon biologique est un échantillon issu dudit patient après le commencement d'un traitement pour l'état ; ou (ii) le deuxième échantillon biologique est un échantillon issu dudit patient une semaine à douze mois après le commencement d'un traitement pour l'état ; ou (iii) le deuxième échantillon biologique est un échantillon issu dudit patient au moins un mois après l'administration de MSC ; ou (iv) le deuxième échantillon biologique est un échantillon issu dudit patient au moins trois mois après l'administration de MSC ; ou (v) à la fois le premier et le deuxième échantillon biologique sont des échantillons issus du patient après le commencement de la thérapie.

11. Le procédé selon l'une quelconque des revendications 1 à 10, sachant que le premier échantillon biologique est un échantillon issu dudit patient avant le commencement d'un traitement pour ledit état et le deuxième échantillon biologique est un échantillon issu dudit patient après le commencement du traitement, sachant que si le niveau déterminé dudit au moins un biomarqueur dans ledit premier et ledit deuxième échantillon est approximativement le même, on détermine qu'une dose supplémentaire de cellules souches mésenchymateuses doit être administrée.

12. Le procédé selon l'une quelconque des revendications 1 à 11, sachant que le procédé comprend en outre la détermination du niveau dudit biomarqueur dans des échantillons biologiques additionnels issus dudit patient, comme un troisième, quatrième, cinquième, etc. échantillon biologique, sachant que chacun desdits échantillons biologiques est issu dudit patient à des moments différents avant et/ou après le commencement d'une thérapie pour l'état.

13. Le procédé selon l'une quelconque des revendications 1 à 12, sachant que l'échantillon biologique est un échantillon de sang circulant ou une fraction de celui-ci.

Figure 1

Figure 2

Figure 3

Figure 4

Figure 5

**Figure 6**

**Figure 7**

**Figure 8**

**Figure 9**

**Figure 10**

Serum MIF Levels

**Figure 11**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- AU 2009001070 W
- WO 2010020005 A
- AU 2009201915
- AU 2013204930
- US 20110212104 A
- US 20140248638 A
- EP 2639296 A1
- WO 9851317 A
- US 2011029357 A
- WO 2013023233 A
- AU 2012001140 W
- WO 2013040649 A
- AU 2012000272 W
- WO 2012122603 A

**Non-patent literature cited in the description**

- **JONG Y. P. et al.** *Nature Immunology,* 2001, vol. 2 (11
- Clinical Immunology. 1991
- Methods in Cell Biology: Antibodies in Cell Biology. 1993, vol. 37
- **ALEXANDER et al.** *Exp Neurol.,* August 2012, vol. 236 (2), 351-362
- **V.B. KRAUS et al.** *Osteoarthritis and Cartilage,* 2011, vol. 19
- **BLABER SP ; WEBSTER RA ; HILL CJ et al.** Analysis of in vitro secretion profiles from adipose-derived cell populations. *J Transl Med,* 2012, vol. 10, 172-88
- *J Orthop Res.,* July 2013, vol. 31 (7), 999-1006